# EUROPÄISCHE PATENTSCHRIFT

(11) **EP 0 684 225 B1**
(45) Veröffentlichungstag und Bekanntmachung des Hinweises auf die Patenterteilung: **23.07.1997**
(21) Anmeldenummer: 95112355.3
(22) Anmeldetag: 24.01.1992
(51) Int. Cl.: C07C 251/48, A01N 37/50, C07C 251/50, C07C 251/52, C07C 251/54, C07C 251/56, C07C 251/58, C07C 251/86, C07C 69/734, C07C 69/653, C07D 333/28, C07D 263/32

(54) **Imino-substituierte Phenylderivate, ihre Herstellung und diese enthaltende Fungizide**
Imino-substituted phenyl derivatives, their preparation and fungicides containing them
Dérivés de phényle iminosubstitués, leur préparation et fongicides les contenant

(30) Priorität: 20.02.1991 DE 4105160
(43) Veröffentlichungstag der Anmeldung: 29.11.1995
(62) Teilanmeldung aus: 92101121.9
(73) Patentinhaber: BASF AKTIENGESELLSCHAFT, 67056 Ludwigshafen (DE)
(72) Erfinder: Grammenos, Wassilios, Dr., D-67063 Ludwigshafen (DE); Harreus, Albrecht, Dr., D-67063 Ludwigshafen (DE); Sauter, Hubert, Dr., D-68167 Mannheim (DE); Hellendahl, Beate, Dr., D-67105 Schifferstadt (DE); Dötzer, Reinhard, Dr., D-69469 Weinheim (DE); Ammermann, Eberhard, Dr., D-64646 Heppenheim (DE); Lorenz, Gisela, Dr., D-67434 Hambach (DE)

(56) Entgegenhaltungen:
- EP-A- 0 254 426
- EP-A- 0 331 061
- EP-A- 0 370 629
- EP-A- 0 415 569

## Beschreibung

Die vorliegende Erfindung betrifft neue substituierte Oximether, Hydrazone und Fungizide, welche diese Verbindungen enthalten.

Es ist bekannt, Phenylacrylsäureenolether als Fungizide (EP 370 629) zu verwenden. Ihre Wirkung ist jedoch unbefriedigend.

Es wurde nun überraschend gefunden, daß substituierte Oximether und Hydrazone der allgemeinen Formel I 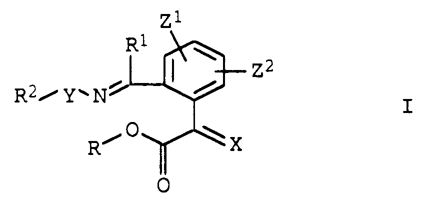 in der die Substituenten folgende Bedeutung haben:
R
   Wasserstoff, C₁-C₆-Alkyl;
R¹
   Wasserstoff, C₁-C₄-Alkyl, C₁-C₄-Halogenalkyl, ggf. substituiertes Aryl;
Z¹, Z²
   gleich oder verschieden Wasserstoff, Halogen, C₁-C₄-Alkoxy, C₂-C₄-Alkenyloxy, Cyano, Nitro, C₁-C₄-Halogenalkyl, C₂-C₄-Halogenalkenyloxy, C₂-C₄-Alkenyl, C₁-C₄-Halogenalkoxy, C₁-C₆-Alkyl oder C₂-C₄-Alkinyl,
Y
   Sauerstoff (-O-), Stickstoff (-NH-) oder ein durch C₁-C₆-Alkyl (R³) substituierter Stickstoff (-N(R³)-);
R²
   Wasserstoff, ggf. substituiertes Alkyl, ggf. substituiertes Cycloalkyl, ggf. substituiertes Alkenyl, ggf. substituiertes Aryl, ggf. substituiertes Alkinyl, ggf. substituiertes Cycloalkenyl, ggf. substituiertes Arylalkyl, ggf. substituiertes Aryloxyalkyl, ggf. substituiertes Heteroaryl, ggf. substituiertes Heteroarylalkyl, ggf. substituiertes Arylalkenyl, ggf. substituiertes Heteroarylalkenyl, ggf. substituiertes Heterocyclyl, ggf. substituiertes Heterocyclyloxy, ggf. substituiertes Arylcarbonyl, ggf. substituiertes Heteroarylcarbonyl oder C₁-C₄-Alkoxycarbonyl-C₁-C₄-alkyl,
   wobei Y und R² einen Ring bilden können, der ggf. substituiert sein kann, sofern Y für -NH- oder -NR³- steht;
X
   CH-C₁-C₄-Alkoxy oder CH-C₁-C₄-Alkylthio;
   eine gute fungizide Wirkung haben.

Außerdem wurde gefunden, daß Verbindungen der Formel I eine gute insektizide, nematizide und akarizide Wirkung haben. Die fungizide Wirkung wird bevorzugt.

Im Hinblick auf ihre biologische Wirksamkeit zur Bekämpfung von Schadpilzen kommen Verbindungen I in Betracht, in denen die Substituenten z.B. die folgende Bedeutung haben:
X
   C₁-C₄-Alkyliden wie Methyliden, Ethyliden, n- oder iso-Propyliden, n-, iso- oder sec.-Butyliden;
   C₁-C₄-Alkoxymethyliden wie Methoxy-, Ethoxy-, n- oder iso-Propoxy-, n-, iso-, sec.- oder tert.-Butoxymethyliden;
   C₁-C₄-Alkylthiomethyliden wie Methyl-, Ethyl-, n- oder iso-Propyl-, n-, iso-, sec.- oder tert.-Butylthiomethyliden;
   C₁-C₄-Alkoxyimino wie Methoxy-, Ethoxy-, n- oder iso-Propoxy-, n-, iso-, sec.- oder tert.-Butoxyimino;
Z¹, Z²
   Halogen wie Fluor, Chlor, Brom und Iod oder z.B. Methyl, Methoxy, Cyano und Nitro;
R, R³
   Wasserstoff;
   C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
R¹
   Wasserstoff;
   C₁-C₄-Alkyl wie Methyl, Ethyl, Propyl, 1-Methylethyl, Butyl, 1-Methylpropyl, 2-Methylpropyl und 1,1-Dimethylethyl;
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
   ggf. substituiertes Aryl wie Phenyl, Naphthyl und Anthryl, bevorzugt Phenyl und Napthyl, insbesondere Phenyl;
R²
   Wasserstoff;
   C₁-C₆-Alkyl wie vorstehend genannt;
   C₁-C₄-Halogenalkyl wie vorstehend genannt;
   ggf. substituiertes C₃-C₁₅-Alkenyl, besonders C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
   ggf. substituiertes C₃-C₈-Alkinyl, besonders C₃-C₆-Alkinyl wie 2-Propinyl, 2-Butinyl, 3-Butinyl, 1-Methyl-2-propinyl, 2-Petninyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
   ggf. substituiertes C₃-C₈-Cycloalkyl wie Cyclopropyl, Cyclobutyl, Cyclopentyl, Cyclohexyl, Cycloheptyl und Cyclooctyl;
   C₁-C₄-Alkoxycarbonyl-C₁-C₄-Alkyl wie Methoxycarbonylmethyl, Ethoxycarbonylmethyl, Propyloxycarbonylmethyl, 1-Methylethyloxycarbonylmethyl, Butyloxycarbonylmethyl, 1-Methylpropyloxycarbonylmethyl und 1,1-Dimethylethyloxycarbonylmethyl;
   ggf. substituiertes C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl;
   ggf. substituiertes Aryl (z.B. Phenyl, Naphthyl, Antrhyl);
   ggf. substituiertes Aryl-C₁-C₄-alkyl (z.B. Benzyl, 1-Phenethyl, 2-Phenethyl, 1-Phenylpropyl, 2-Phenylpropyl, 3-Phenpropyl, 2-Methyl-3-Phenylpropyl, 2-Methyl-2-Phenylpropyl, 4-Phenylbutyl);
   ggf. substituiertes Acyl, z.B. C₁-C₄-Alkylcarbonyl wie Acetyl, Propionyl, Butyryl, Phenylacetyl und Chloracetyl;
   ggf. substituiertes Arylcarbonyl wie Benzoyl und 2,4-Dichlorbenzoyl;
   ggf. substituiertes Heteroarylcarbonyl wie 2-Pyridylcarbonyl;
   ggf. substituiertes Aryl-C₁-C₄-alkenyl (z.B. Phenyl-1-ethenyl, 2-Phenyl-1-propenyl, 2,2-Diphenylethenyl, 1-Phenyl-1-propen-2-yl und 1-Phenylbutenyl);
   ggf. substituiertes Aryloxy-C₁-C₄-Alkyl wie Phenoxymethyl, Phenoxyethyl und Phenoxypropyl;
   ggf. substituiertes Heteroaryl (z.B. 2-Pyridyl, 3-Pyridyl, 4-Pyridyl, 3-Pyridazinyl, 4-Pyridazinyl, 1-Pyrazolyl, 3-Pyrazolyl, 4-Pyrazolyl, 2-Pyrazinyl, 2-Pyrimidinyl, 4-Pyrimidinyl, 5-Pyrimidinyl, 1-Indazolyl, 3-Indazolyl, 2-Furyl, 3-Furyl, 1-Pyrrolyl, 2-Pyrrolyl, 3-Pyrrolyl, 2-Thiophenyl, 3-Thiophenyl, 1-Imidazolyl, 2-Imidazolyl, 4-Imidazolyl, 5-Imidazolyl, 2-Isoindolyl, 1-Indolyl, 1-Indazolyl, 1,2-Benzothiazol-3-yl, 1,3-Benzothiazol-2-yl, 3-Isoaxazolyl, 4-Isoocazolyl, 5-Isoazolyl, 3-Isothiazolyl, 4-Isothiazolyl, 5-Isothiazolyl, 1,2-Benzooxazol-3-yl, 1,3-Benzoxazol-2-yl, 1,2,4-Triazolyl, 1,3,4-Triazolyl, 7-Purinyl, 2-Chinonyl, 3-Chinonyl, 4-Chinonyl, 1-Isochinolyl, 3-Isochinolyl, 4-Isochinolyl, 2-Benzofuranyl, 3-Benzofuranyl, 1-Isobenzofuranyl, 1,2,4-Triazin-3-yl und 1,3,5-Triazin-2-yl;
   ggf. substituiertes Heteroaryl-C₁-C₄-alkyl (z.B. 2-Pyridylmethyl, 3-Pyridylmethyl);
   ggf. substituiertes Heteroaryl-C₂-C₄-alkenyl (z.B. 2'-Furyl-2-ethenyl, 2'-Thienyl-2-ethenyl, 3'-Pyridyl-2-ethenyl);
   ggf. substituiertes Heterocyclyl (z.B. Oxiranyl, 1-Aziridinyl, 1-Azetidinyl, 1-Pyrrolidinyl, 2-Tetrahydrofuryl, 2-Tetrahydropyranyl, 3-Tetrahydropyranyl, 1-Piperidinyl, 1-Morpholinyl, 1-Piperazinyl, 1,3-Dioxanyl, 3-Tetrahydrothiopyranyl).

Die mit "ggf. substituiert" bezeichneten Reste enthalten neben Wasserstoff z.B. die folgenden Reste:
Halogen wie Fluor, Chlor, Brom und Iod;
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
C₁-C₆-Alkyloxy wie Methyloxy, Ethyloxy, Propyloxy, 1-Methylethyloxy, Butyloxy, 1-Methylpropyloxy, 2-Methlypropyloxy, 1,1-Dimethylethyloxy, Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropyloxy, 1-Ethylpropyloxy, Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
C₁-C₄-Halogenalkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₂-C₆-Alkenyloxy wie Ethenyloxy, 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-2-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy;
Cyano; Cyanato, Thiocyanato; Nitro; Amino; Hydroxy; Carboxyl;
C₁-C₆-Alkylamino wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino;
Di-C₁-C₆-alkylamino, besonders Di-C₁-C₄-alkylamino wie N,N-Dimethylamino, N,n-Diethylamino, N,N-Dipropylamino, N,N-Di-(1-methylethyl)amino, N,N-Di-butylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino; N-(1,1-Di-methylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
Phenyl, Phenoxy, Phenylthio und Phenylamino,
welche ihrerseits ein bis 5 Halogenatome wie vorstehend genannt;
und/oder eine bis fünf C₁-C₆-Alkoxygruppen wie vorstehend genannt;
und/oder eine bis fünf C₁-C₆-Alkylthiogruppen wie vorstehend genannt;
und/oder eine bis fünf C₁-C₄-Halogenalkylgruppen wie vorstehend genannt;
und/oder eine bis vier C₁-C₆-Alkoxyiminomethylgruppen wie Methoxyiminomethyl, Ethoxyiminomethyl, n-Propoxyiminomethyl, n-Butoxyiminomethyl, n-Pentoxyiminomethyl, n-Hexoxyiminomethyl, Allyloxyiminomethyl, Benzyloxyiminomethyl, iso-Propoxyiminomethyl, iso-Butoxyiminomethyl und tert.-Butoxyiminomethyl,
und/oder eine bis vier C₁-C₆-Alkoxyiminoethylgruppen wie Methoxyiminoethyl, Ethoxyimino-1-ethyl, n-Propoxyimino-1-ethyl, n-Butoxyimino-1-ethyl, n-Pentoxyimino-1-ethyl, n-Hexoxyimino-1-ethyl, Allyloxyimino-1-ethyl, Benzyloxyimino-1-ethyl; und/oder Phenyl, Phenoxy und Benzyloxy;
und/oder eine C₁-C₄-Alkyliminomethylgruppe wie Methyliminomethyl und Ethyliminomethyl;
und/oder eine bis vier C₃-C₆-Cycloalkylgruppen wie Cyclopropyl, Cyclobutyl, Cyclopentyl und Cyclohexyl, tragen können.

Die neuen Verbindungen der allgemeinen Formel I können bei der Herstellung aufgrund der C=C bzw. C=N-Doppelbindungen als E/Z-Isomerengemische anfallen. Diese können in üblicher Weise durch Kristallisation oder Chromatographie in die einzelnen Komponenten aufgetrennt werden.

Sowohl die einzelnen Isomeren Verbindungen als auch ihre Gemische werden von der Erfindung umfaßt und sind als Fungizide brauchbar.

Die Herstellung der Verbindungen der allgemeinen Formel I gemäß Anspruch 1 erfolgt beispielsweise wie in Schema 1 beschrieben (Z¹, Z² gleich H).

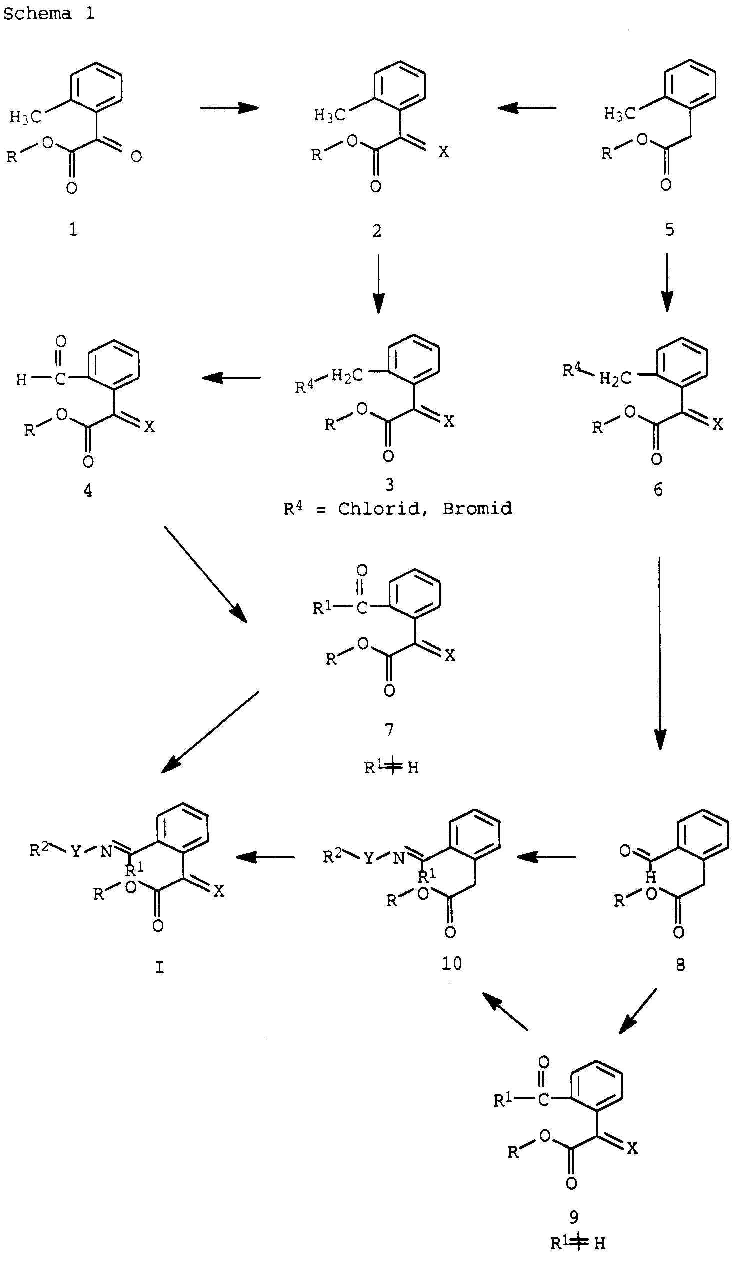

Die Verbindungen der allgemeinen Formel I, in denen X CH-Alkoxy ist, lassen sich beispielsweise aus den Ketoestern 1 durch Wittig- oder Wittig-Horner-Reaktion herstellen (vgl. EP 348 766, DE 37 05 389, EP 178 826).

Für Verbindungen der Formel I, in der X=CH-S-Alkyl ist, kann die Herstellung nach den Methoden aus EP 244 077 oder 310 954 erfolgen.

Die Zwischenprodukte der Formeln 3 und 6 lassen sich aus den Verbindungen 2 und 5 herstellen, indem man diese nach bekannten Methoden halogeniert, z.B. mit Chlor, Brom, N-Bromsuccinimid in einem inerten Lösungsmittel (z.B. CCl₄, Cyclohexan) unter Belichtung mit z.B. einer Hg-Dampflampe oder mit Radikastartern wie z.B. Dibenzoylperoxid.

Benzaldehyde der Formeln 4 und 8 sind entweder bekannt (X=CHOCH₃, vgl. EP 88 300 280) oder können durch Oxidation der Benzylhalogenide 3 oder 6 z.B. mit N-Methylmorpholin-N-Oxid in einem inerten Lösungsmittel wie z.B. CCl₄ oder Cyclohexan hergestellt werden. Außerdem kann man die Benzaldehyde der Formeln 4 und 8 herstellen, wenn man die entsprechenden Benzalhalogenide mit einem Oxidationsmittel wie z.B. Silbernitrat in Methanol oder Ethylenglykolmonomethylether [vgl. J. Am. Chem. Soc. 78, 1689 (1956)] behandelt.

Die Herstellung der Verbindungen der allgemeinen Formeln 7 und 9 kann beispielsweise wie in Schema 2 beschrieben erfolgen.

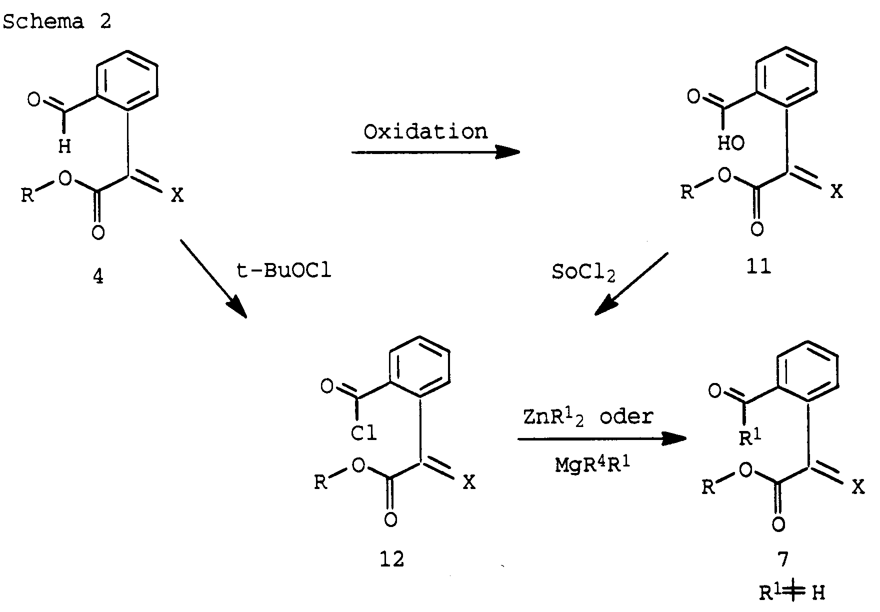

Die Benzaldehyde 4 und 8 können in bekannter Weise in die Carbonsäuren 11 und 13 umgewandelt werden [vgl. Organikum 15. Auflage, 447B (1977)].

Aus den so erhaltenen Carbonsäuren 11 und 13 lassen sich in an sich bekannter Weise die Säurechloride 12 und 14 herstellen [vgl. Organikum 15. Auflage, 526 fB (1977)].

Alternativ können die Säurechloride 12 und 14 auch direkt aus den Bezaldehyden 4 und 8 erhalten werden, wenn man sie beispielsweise mit t-BuOCl in einem inerten Lösungsmittel wie z.B. CCl₄ behandelt [vgl. D. Ginsburg, J. Amer. Chem. Soc. 73, 702 (1951)]. Die Ketone 7 und 9 erhält man beispielsweise aus den Säurechloriden 12 und 14 durch Umsetzung mit zinkorganischen Verbindungen vom Typ ZnR¹₂ analog (Org. React. 1954, 8, 28).

Die Darstellung der neuen Verbindungen der allgemeinen Formel I kann so erfolgen, daß man die neuen Carbonylverbindungen der Formel 7 mit einem substituierten Hydroxylamin der allgemeinen Formel 15 oder Hydrazin der allgemeinen Formel 16 oder mit einem Säureadditionssalz (z.B. Hydrochlorid, Hydrobromid) von 15 oder 16 umsetzt.

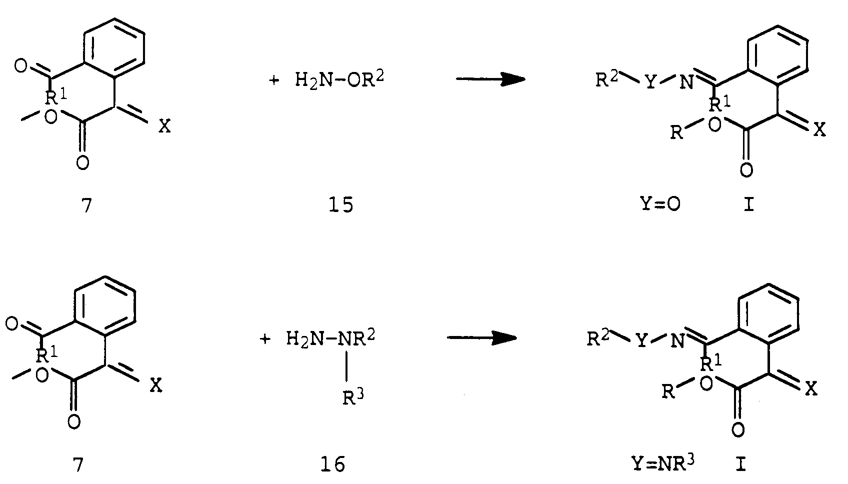

Die Reaktion kann in einem inerten Lösungs- oder Verdünnungsmittel (z.B. Methanol, Ethanol, Toluol) oder in einem Zweiphasensystem (z.B. Toluol/Wasser) durchgeführt werden. Außerdem kann es von Vorteil sein, der Reaktionsmischung eine Base (z.B. Triethylamin, Natriumcarbonat, Kaliumcarbonat, Natriumhydrogencarbonat, Kaliumhydrogencarbonat, Natriumhydroxid, Kaliumhydroxid) zuzusetzen.

Alternativ kann bei der Herstellung von I auch wie folgt vorgegangen werden (Schema 4, Z¹, Z² = H):

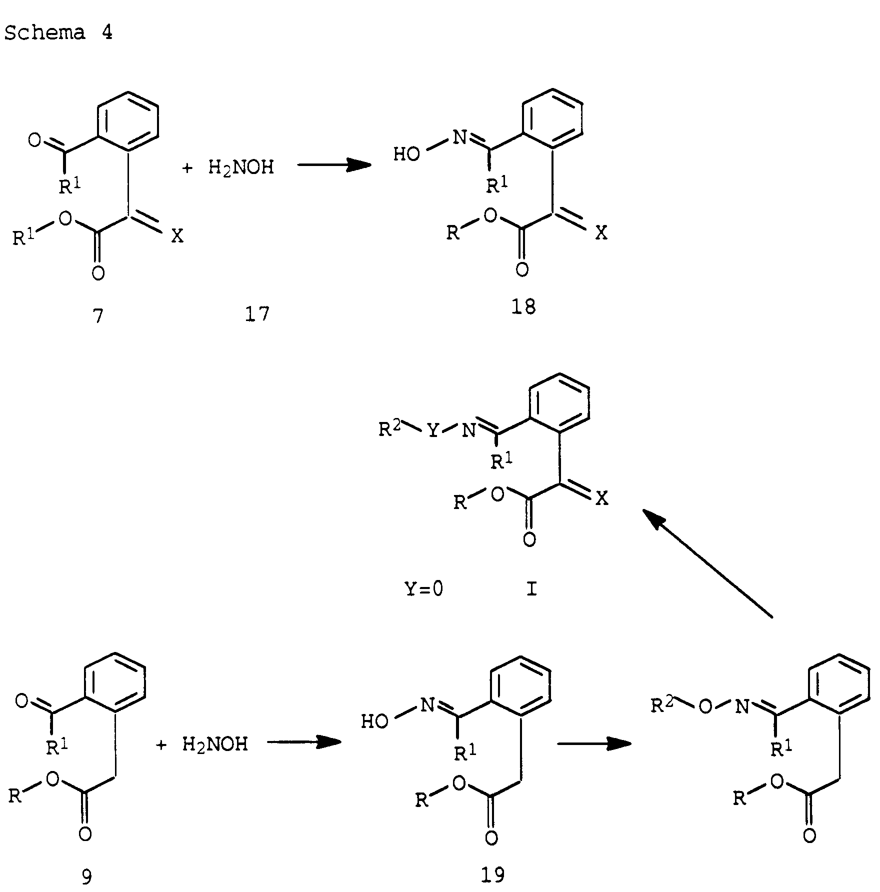

Die zur Herstellung der Verbindungen der allgemeien Formel I (Y=0) benötigten Oxime 18 und 19 (vgl. Schema 4) können aus den entsprechenden Carbonylverbindungen 7 und 9 in bekannter Weise (vgl. Houben-Weyl, Bd. 10/4 (1968)) durch Umsetzung mit dem Hydroxylamin 17 hergestellt werden.

Die Reaktion kann in einem inerten Lösungs- oder Verdünnungsmittel (z.B. Methanol, Ethanol, Toluol) oder in einem Zweiphasensystem (z.B. Toluol/Wasser) durchgeführt werden. Außerdem kann es von Vorteil sein, der Reaktionsmischung eine Base (z.B. Triethylamin, Matriumcarbonat, Kaliumcarbonat, Natriumhydrogencarbonat, Kaliumhydrogencarbonat, Natriumhydroxid, Kaliumhydroxid) zuzusetzen.

Die nachfolgende Alkylierung der Oxime 18 und 19 erfolgt in bekannter Weise mit einer Base (wie z.B. NaOMe, NaH, KOtBu in einem Lösungsmittel wie z.B. Diethylether, Toluol, Dimethylformamid etc.) (vgl. DE 36 23 921).

Hydroxylamine der allgemeinen Formel 15 (H₂N-OR²) sind entweder bekannt oder können nach bekannten Methoden analog Houben-Weyl X/1, 1192, 1183 hergestellt werden.

Hydrazinderivate der allgemeinen Formel 16 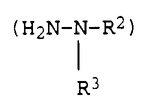 sind entweder bekannt oder können ebenso nach bekannten Methoden analog Houben-Weyl X/2, S. 271, 280, 740 hergestellt werden.

Ein weiterer Zugang zu den Verbindungen der allgemeinen Formel I wird in Schema 5 beschrieben (Z¹, Z² = H, R⁵ = Methyl, Ethyl).

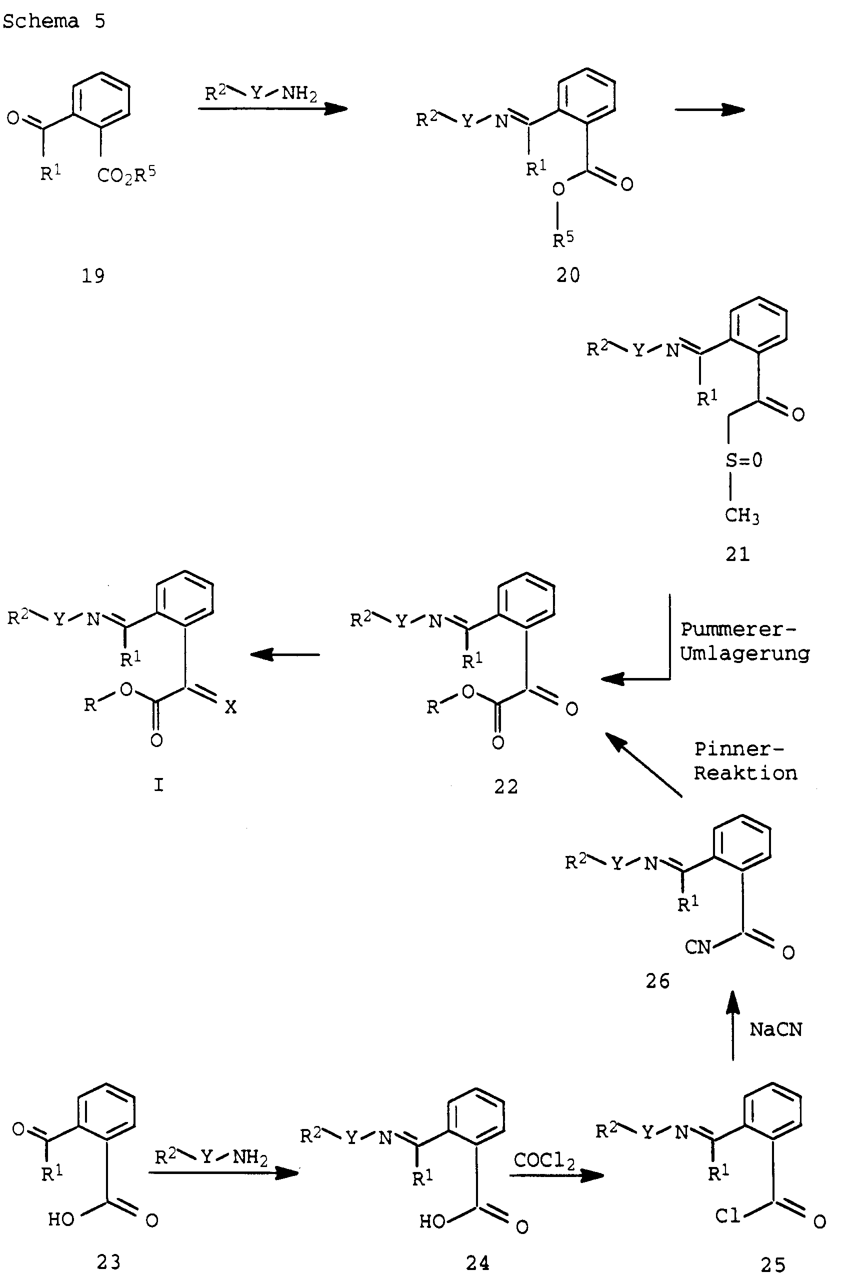

Ausgehend von den Carbonsäureestern der Formel 20 kann man durch Anlagern von Dimethylsulfoxid in Anwesenheit einer starken Base (wie z.B. NaOCH₃, KOtBu) die β-Ketosulfoxide 21 erhalten (vgl. J. Amer. Chem. Soc. 88, 5498 (1966)), die nach Bromierung und anschließendem Verkochen mit einem Alkohol (wie z.B. Methanol, Ethanol) in Gegenwart einer Säure in einer sogenannten "Pummerer-Umlagerung" die α-Ketoester 22 ergeben (Synthesis, 47 (1982)).

Alternativ kann man die α-Ketoester der Formel 22 durch Anwendung der sogenannten "Pinner-Reaktion" bei den Acylcyaniden der Formel 26 erhalten (vgl. S. Hünig et al. Ang. Chemie 94, 1 (1982)).

Die Überführung der Carbonsäuren 24 in die Acylcyanide 26 erfolgt in üblicher, an sich bekannter Weise (vgl. "Organikum": 16. Auflage, Berlin 1986, S. 423f, und J.M. Photis, Tetr. Lett. 21, 3539-3540 (1980)).

Die Zwischenprodukte der allgemeinen Formel 19 kann man ausgehend von o-Hydroxyphenylketonen der Formel 27 analog Tetr. Letters, 6781 (1990) (Schema 6) herstellen.

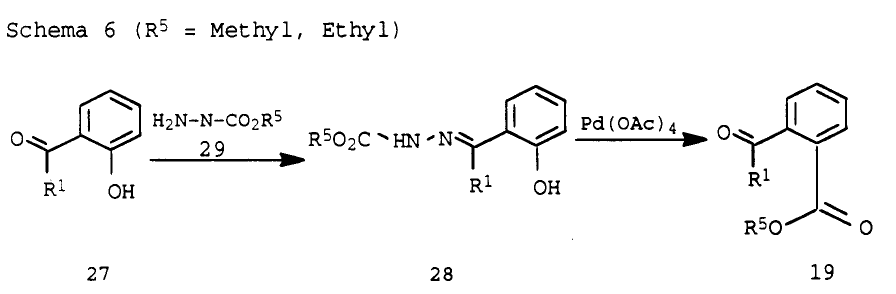

Die folgenden Vorschriften und Beispiele sollen die Herstellung der neuen Wirkstoffe und der neuen Zwischenprodukte erläutern.

### Vorschrift 1

### Herstellung von α-(2-Formylphenyl)-β-methoxy-acrylsäuremethylester

35,5 g (0,125 mol) α-(2-Brommethylphenyl)-β-methoxy-acrylsäuremethylester werden in 500 ml CCl₄ gelöst. Man gibt 43,4 g (0,321) mol) Methylmorpholin-N-oxid-monohydrat zu und erhitzt 20 Stunden unter Rückfluß. Der Feststoff wird abfiltriert und die CCl₄-Phase mit Wasser, mit 2 N Salzsäure und wieder mit Wasser gewaschen, getrocknet und eingeengt. Man erhält 18,5 g (67 %) Produkt als rotbraunes Öl.
¹H-NMR (CDCl₃): 3,7 (s, 3H); 3,8 (s, 3H); 7,2-7,6 (m, 4H); 7,9 (m, 1H) 10,0 ppm (s, 1H).

### Vorschrift 3

### Herstellung von 1-Brom-2-methoxymethyl-benzol

685 g (2,74 mol) o-Brombenzylbromid werden zusammen mit 2,74 mol einer 30 %igen Natriummethylat-Lösung in Methanol 15 Stunden unter Rückfluß erhitzt. Nach dem Abkühlen wird eingeengt, in Essigester aufgenommen und mit Wasser gewaschen. Nach dem Trocknen der organischen Phase über Natriumsulfat wird eingeengt. Man erhält 478,2 g (87 %) Produkt als farblose Flüssigkeit.
¹H-NMR (CDCl₃): 3,5 (s, 3H); 4,58 (s, 2H); 7,08-7,6 ppm (m, 4H).

### Vorschrift 4

### Herstellung von 2-Methoxymethyl-phenylglyoxylsäuremethylester

Zu einer aus 12,0 g (0,46 mol) Magnesiumspänen und 99,5 g (0,5 mol) 1-Brom-2-methoxymethyl-benzol in 500 ml THF (Tetrahydrofuran) hergestellten Grignardlösung werden 500 ml etherische 1 N Zinkchloridlösung zugetropft. Anschließend wird 1 Stunde unter Rückfluß erhitzt und die Lösung von 61,2 g (0,5 mol) Oxalsäuremethylesterchlorid in 100 ml THF bei einer Innentemperatur von -10°C zugetropft. Man läßt auf Raumtemperatur (20°C) kommen, rührt noch 20 Stunden bei Raumtemperatur weiter und hydrolyisert mit Ammoniumchloridlösung. Nach dem Extrahieren mit Ether werden die vereinigten Etherphasen mit Wasser gewaschen, getrocknet und eingeengt. Man erhält 60 g Produkt (57,7 %) als eine leicht bewegliche Flüssigkeit.
¹H-NMR (CDCl₃): 3,38 (s, 3H); 3,98 (s, 3H); 4,8 (s, 2H); 7,3-7,7 ppm (m, 4H).

### Beispiel 2

### Herstellung von 2-[β-Methoxy(acrylsäuremethylester)benzaldehydphenylhydrazon

Zu einer Lösung von 1,5 g (14 mmol) Phenylhydrazin in 20 ml Methanol tropft man bei 25°C die Lösung von 3,5 g (16 mmol) α-(2-Formylphenyl)-β-methoxy-acrylsäuremethylester in 5 ml Methanol und rührt das Reaktionsgemisch 20 Stunden bei 25°C weiter. Dann wird das Produkt abgesaugt, mit Ether sorgfältig nachgewaschen und getrocknet. Man erhält 3,4 g (79 %) der obengenannten Verbindung in Form hellbeiger Kristalle (Verbindung Nr. 5.20, Tabelle V).
Fp: 210°C (Zersetzung)
¹H-NMR (DMSO): 3,65 (s, 3H); 3,85 (s, 3H); 7,8 (s, 1H); 7,8 (m, 1H); 6,75-7,95 (m, 9H); 10,45 ppm (s, 1H).

In entsprechender Weise können die in den nachfolgenden Tabellen aufgeführten Verbindungen hergestellt werden.

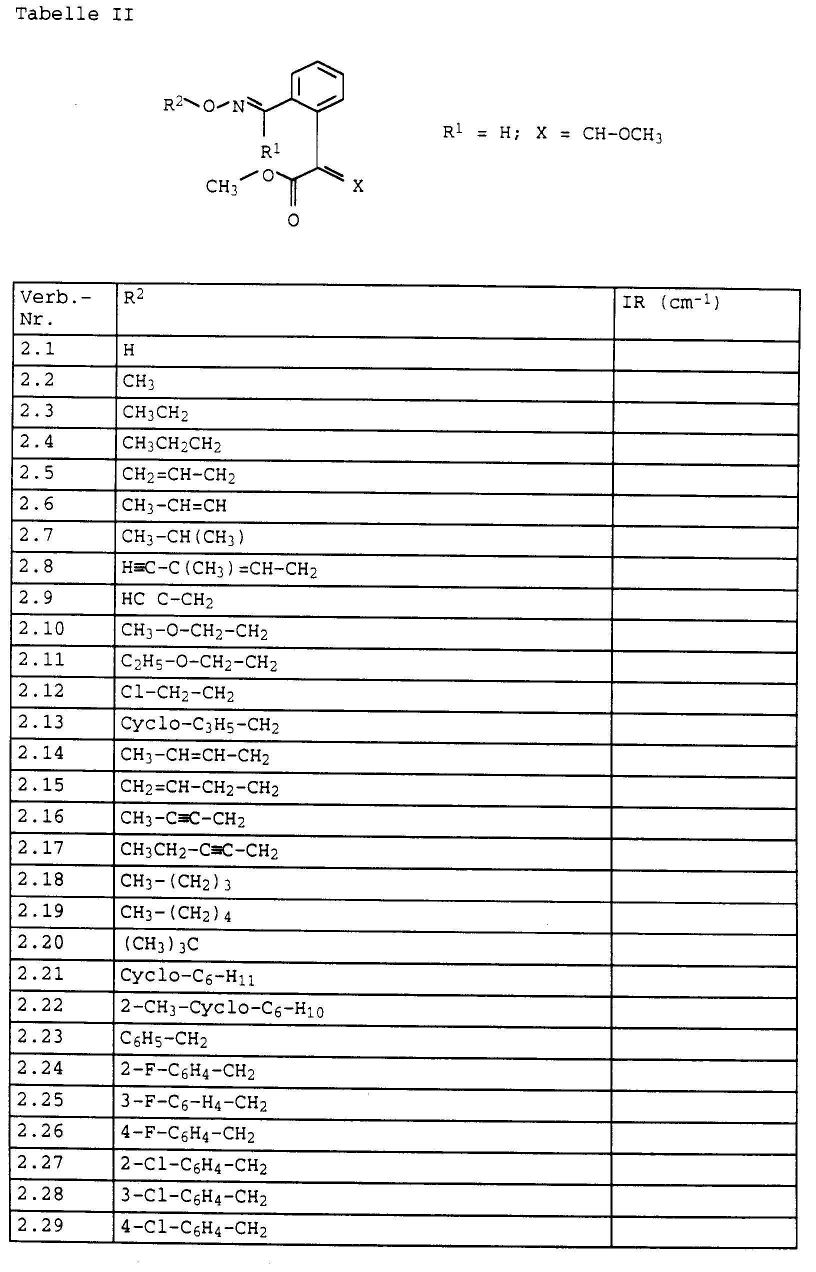

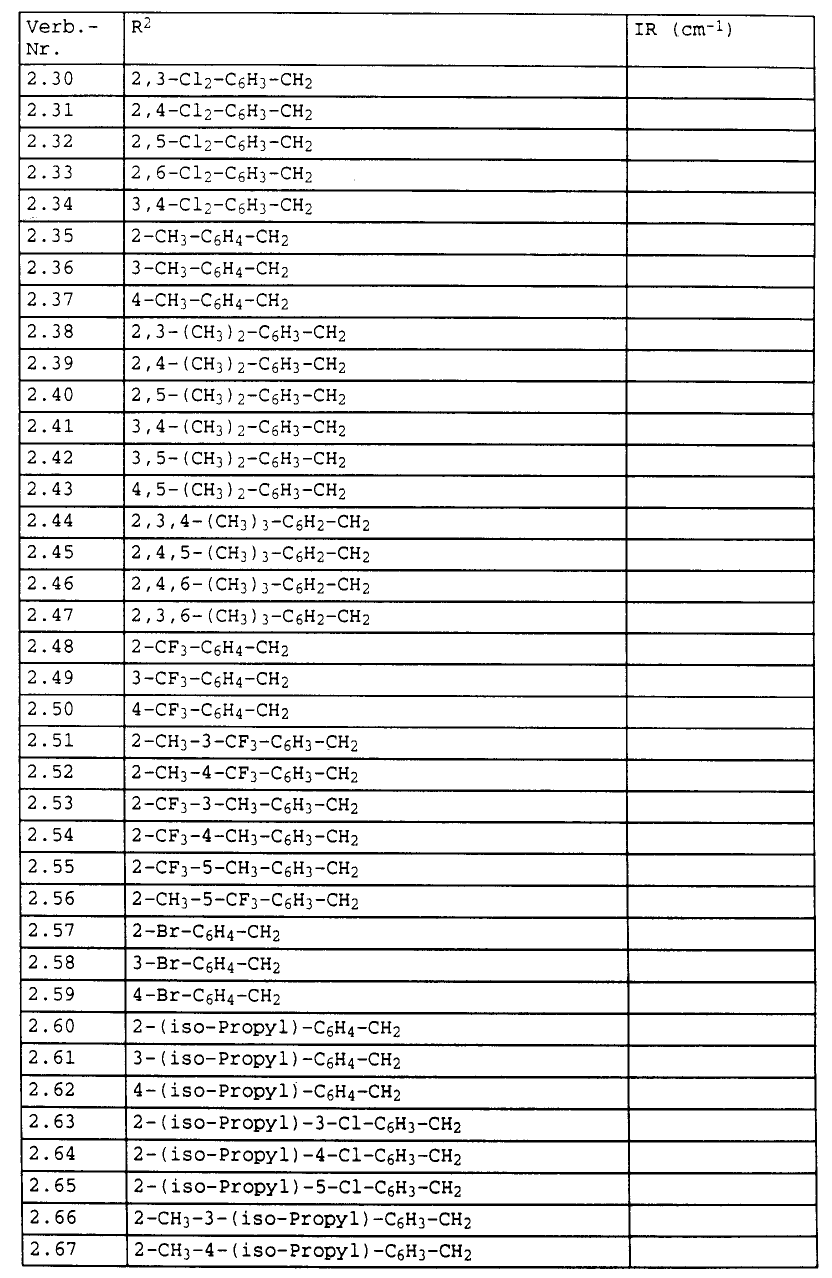

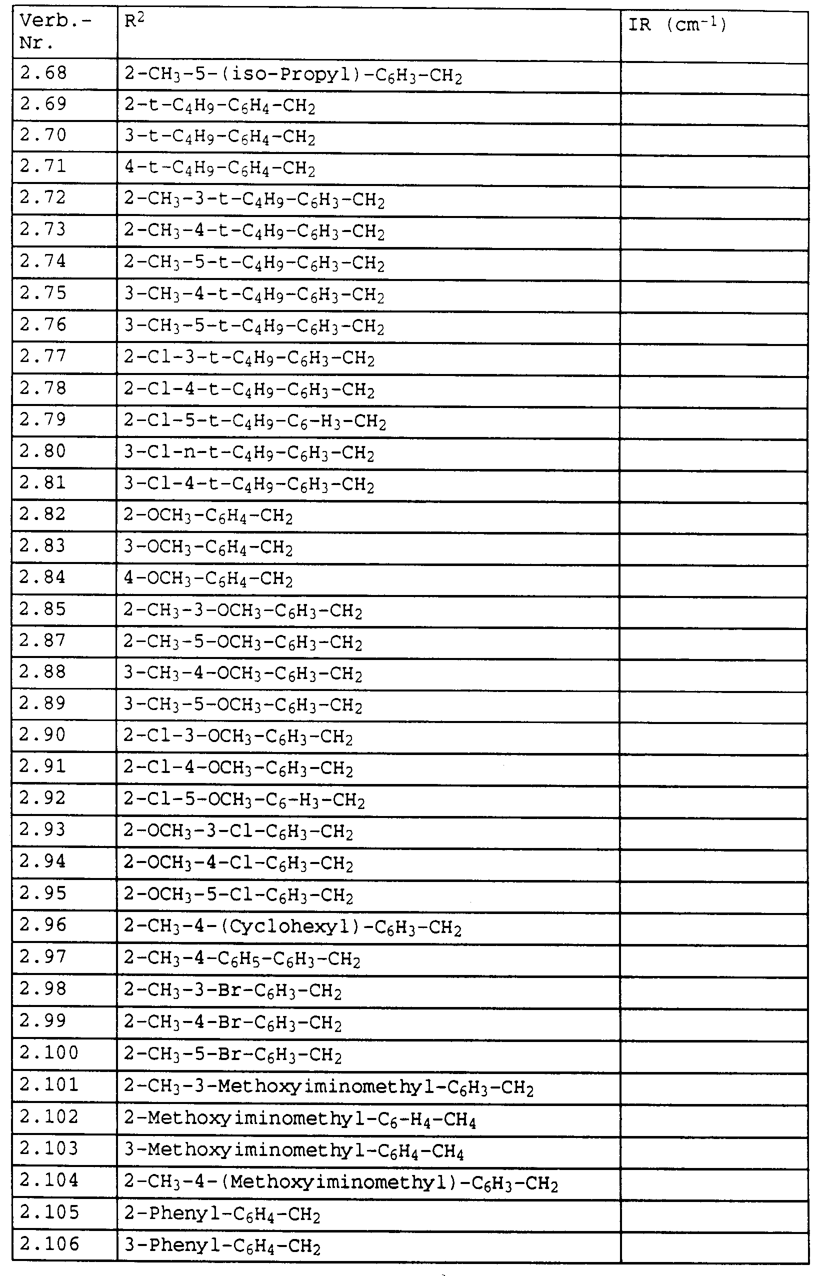

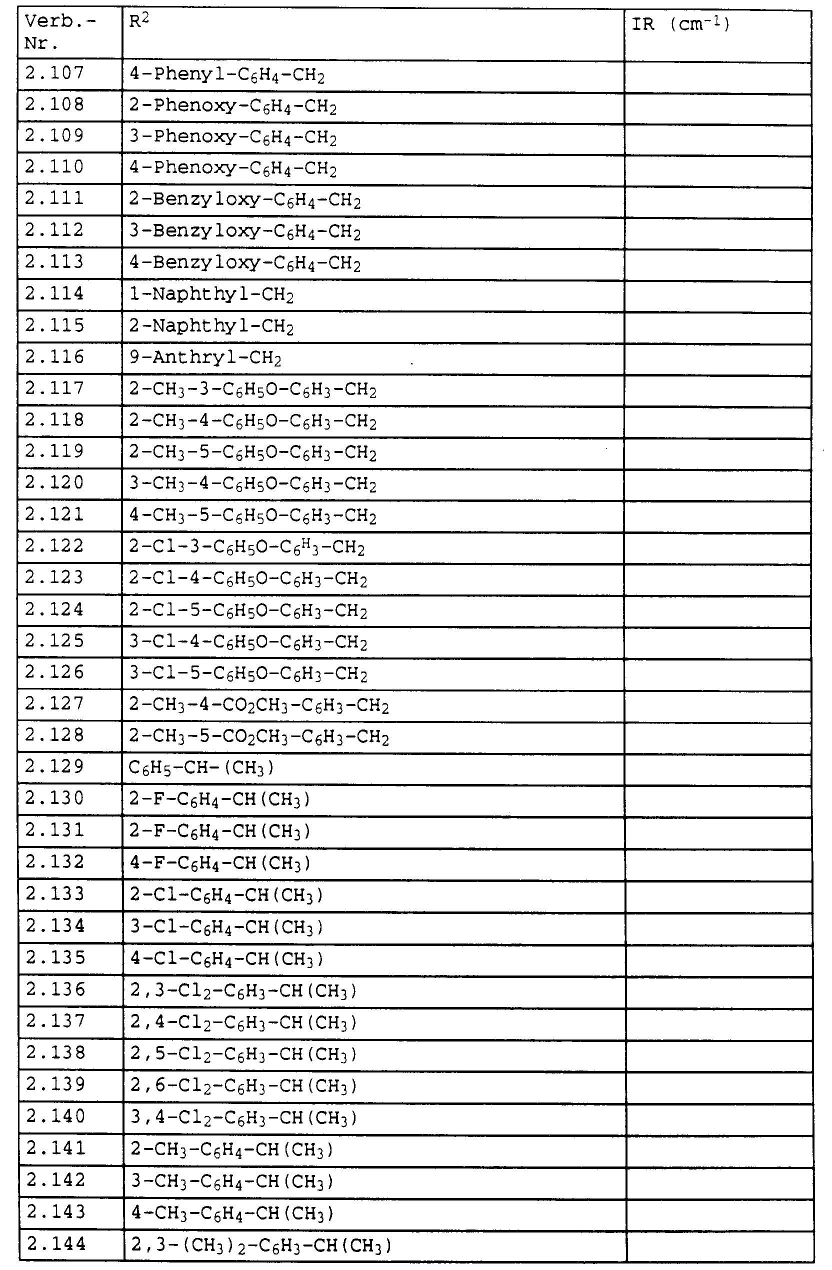

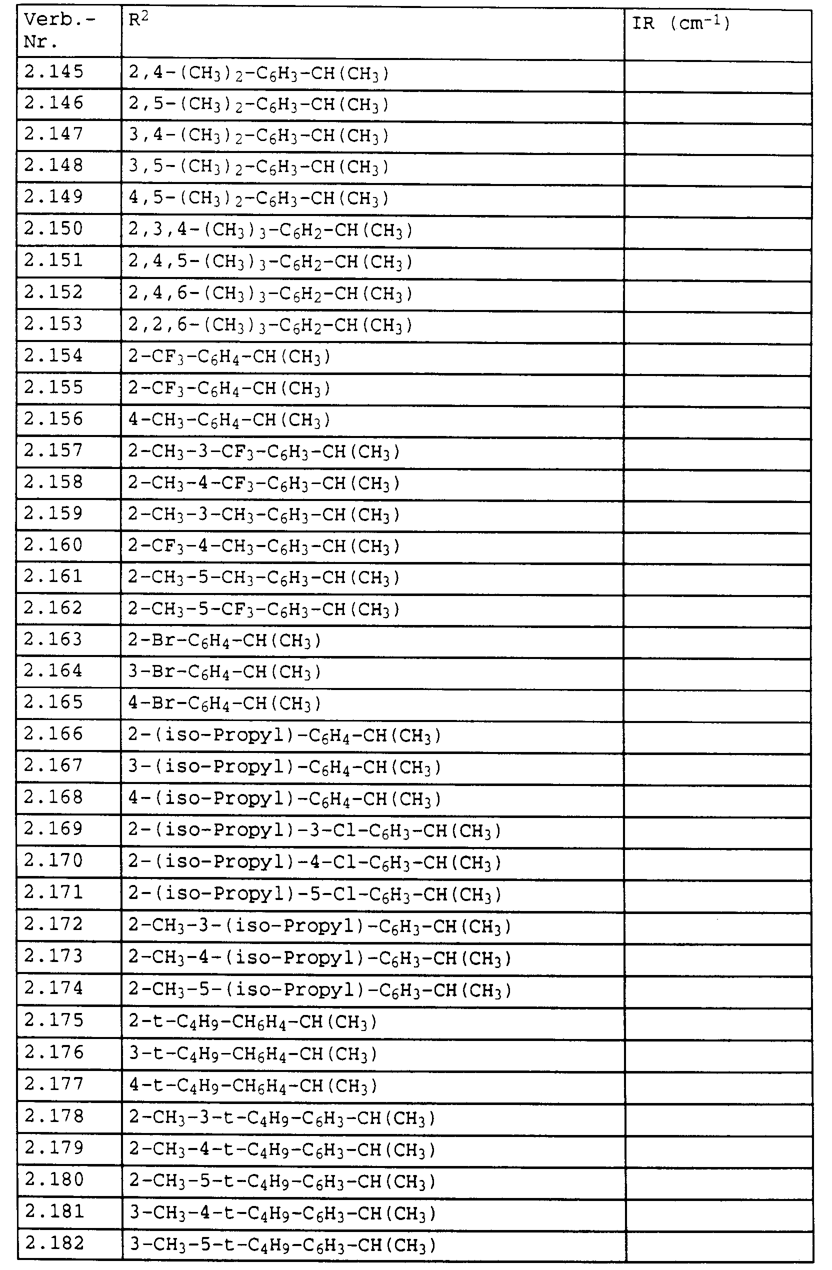

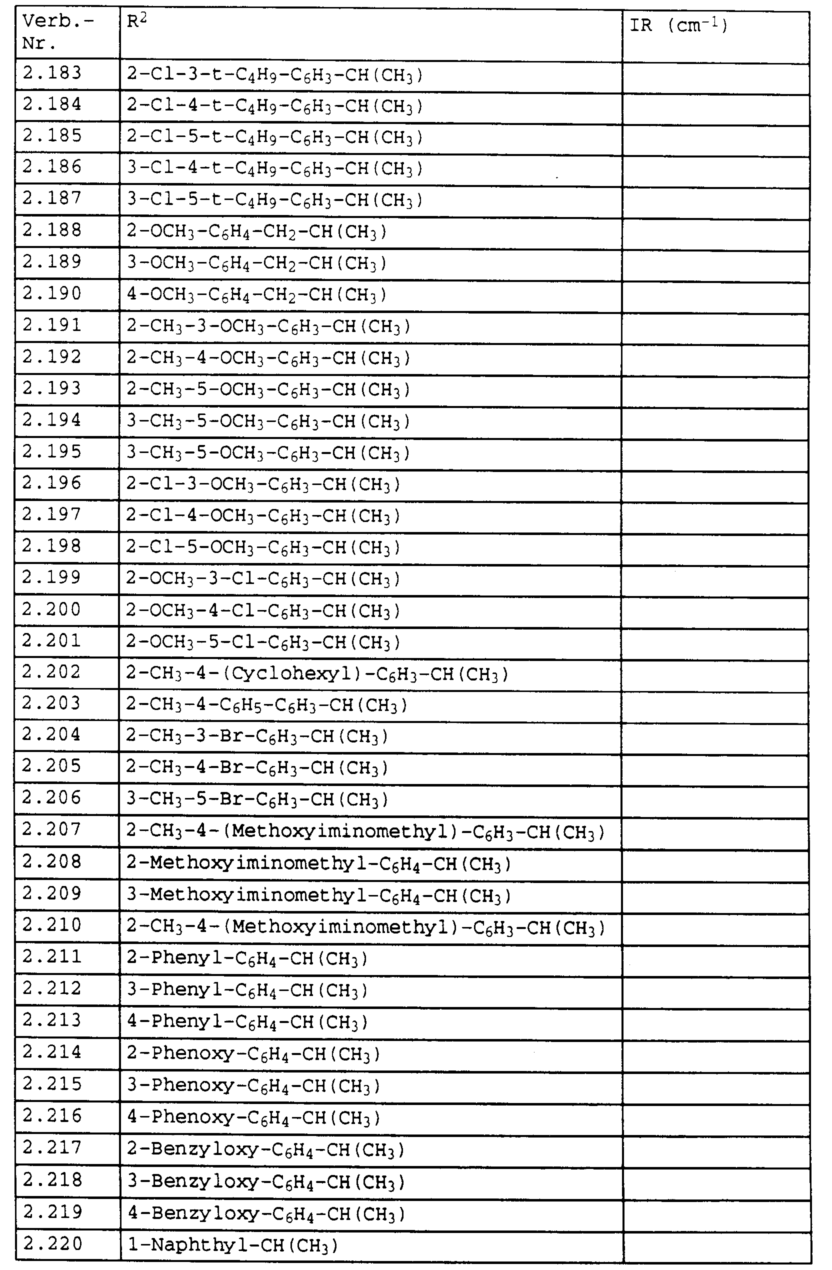

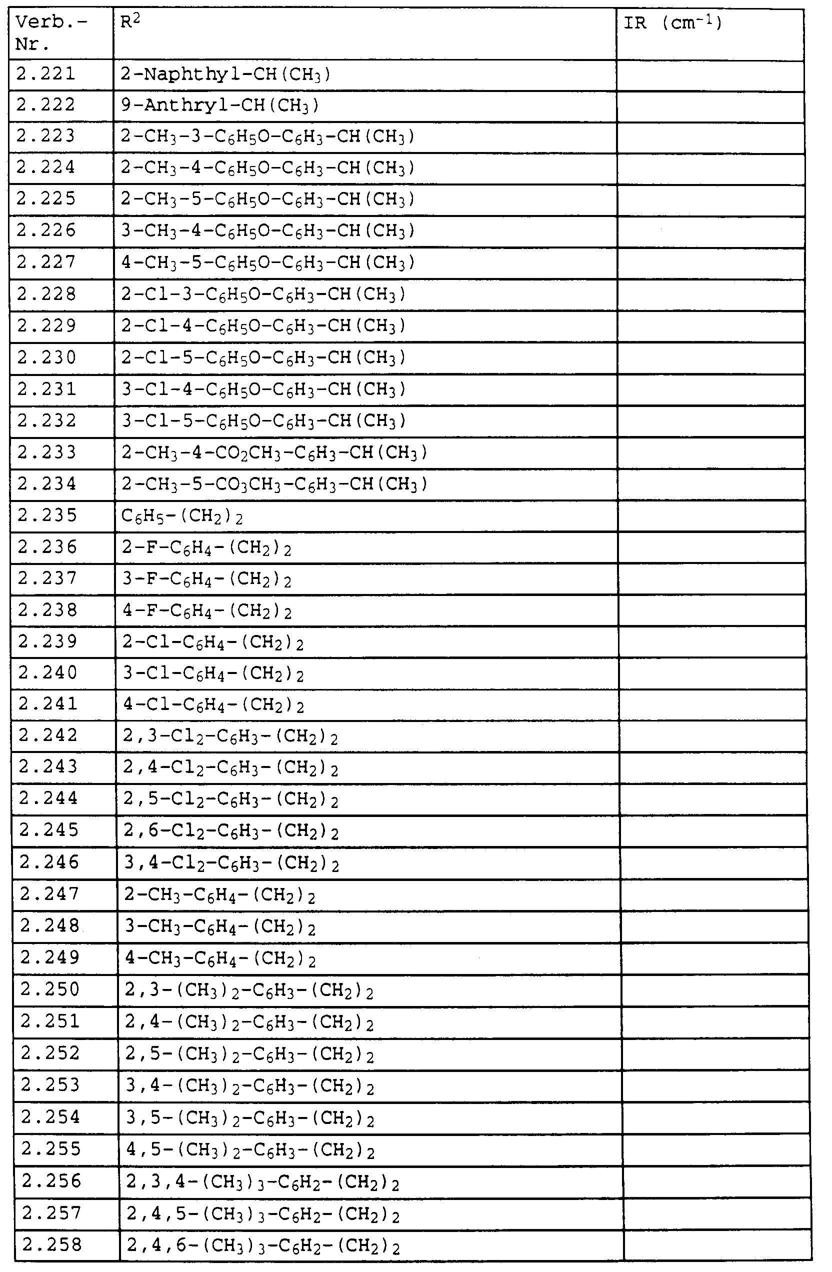

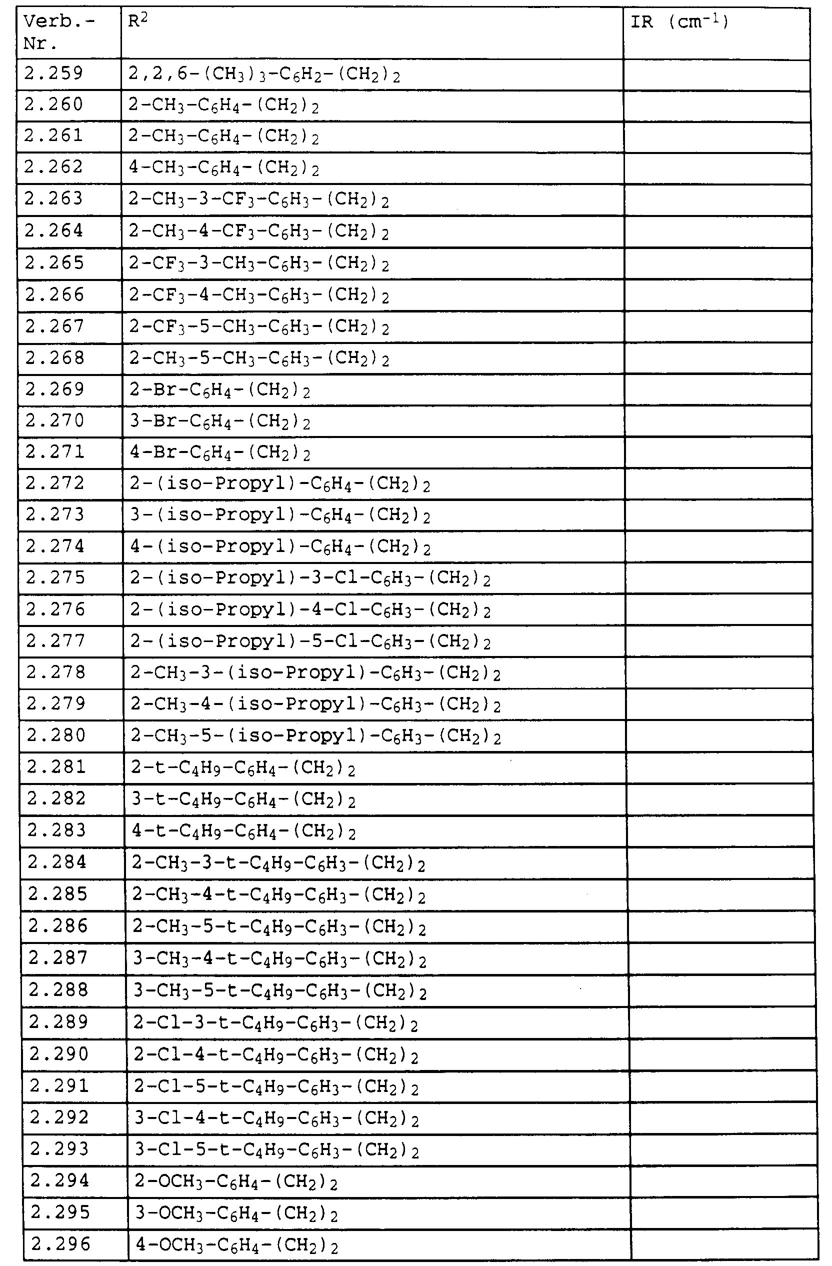

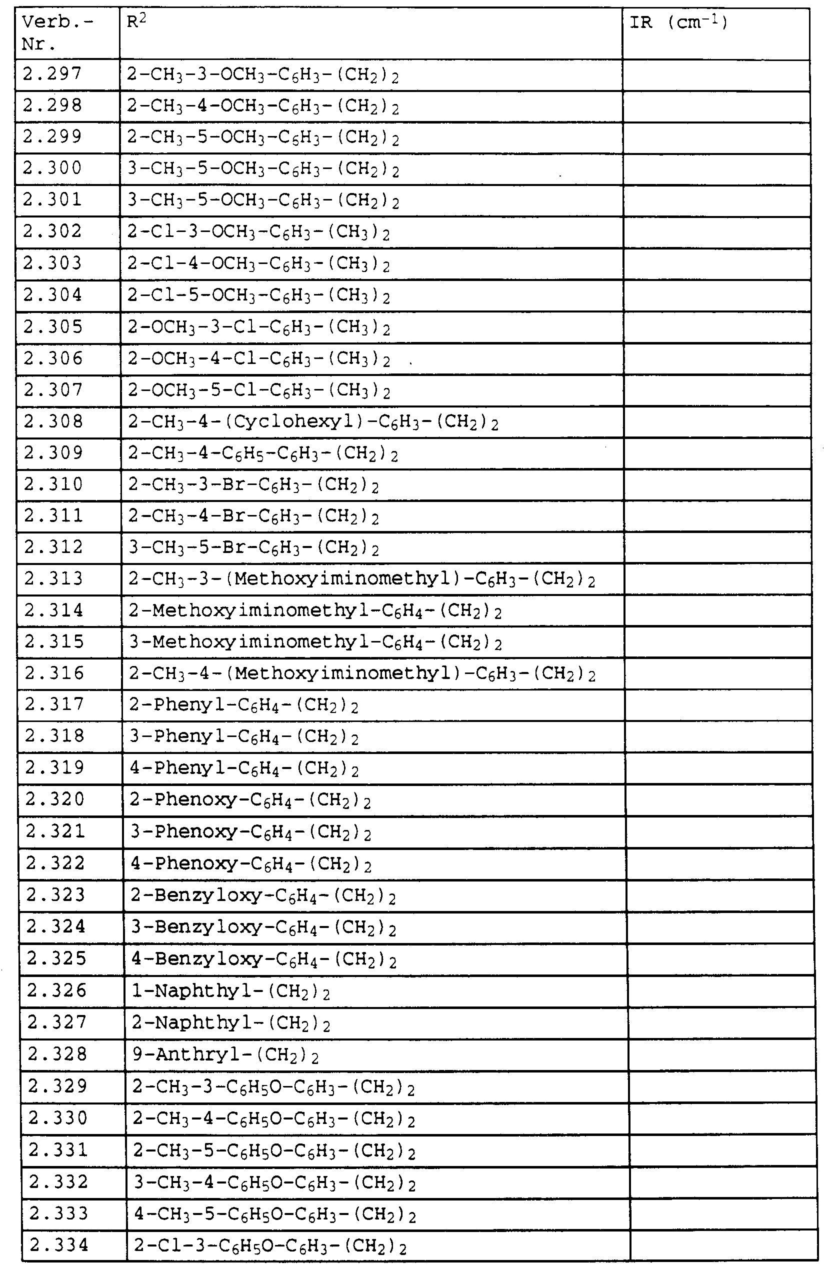

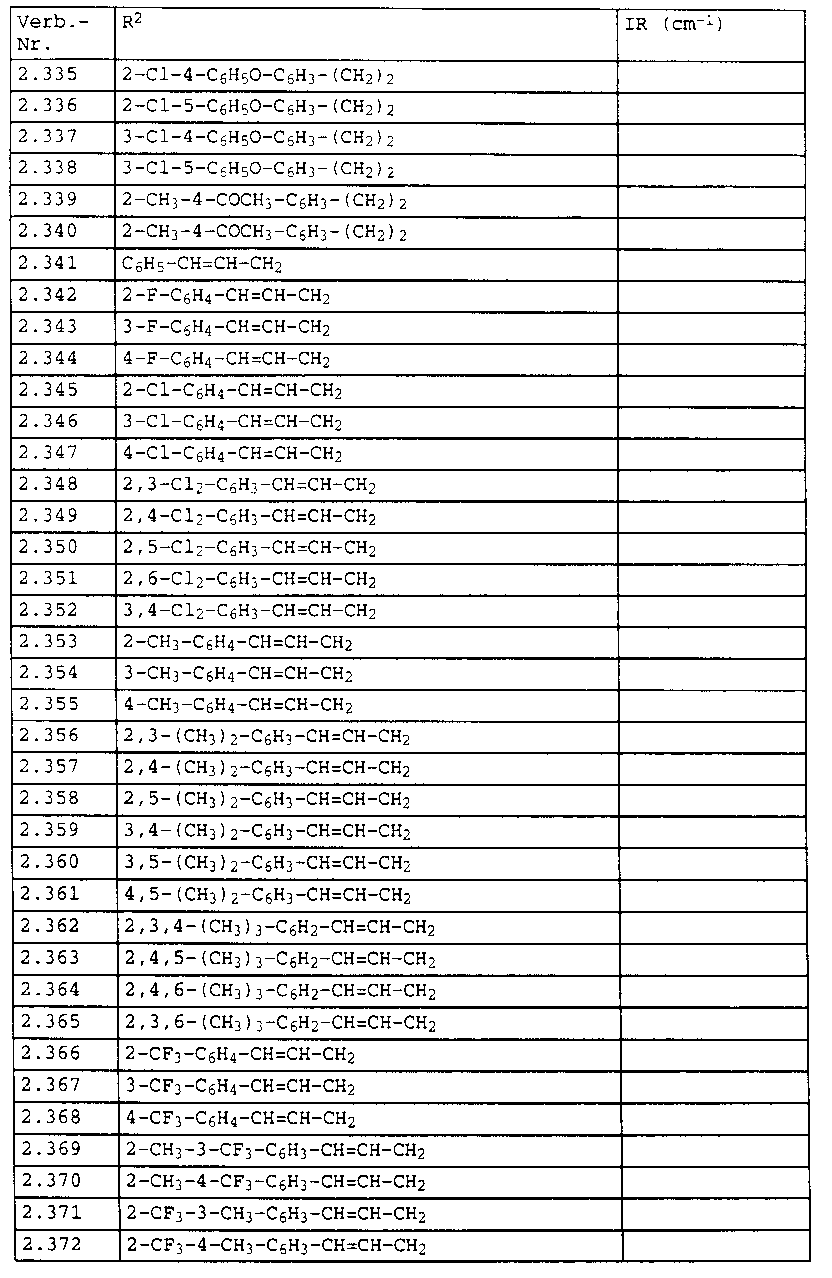

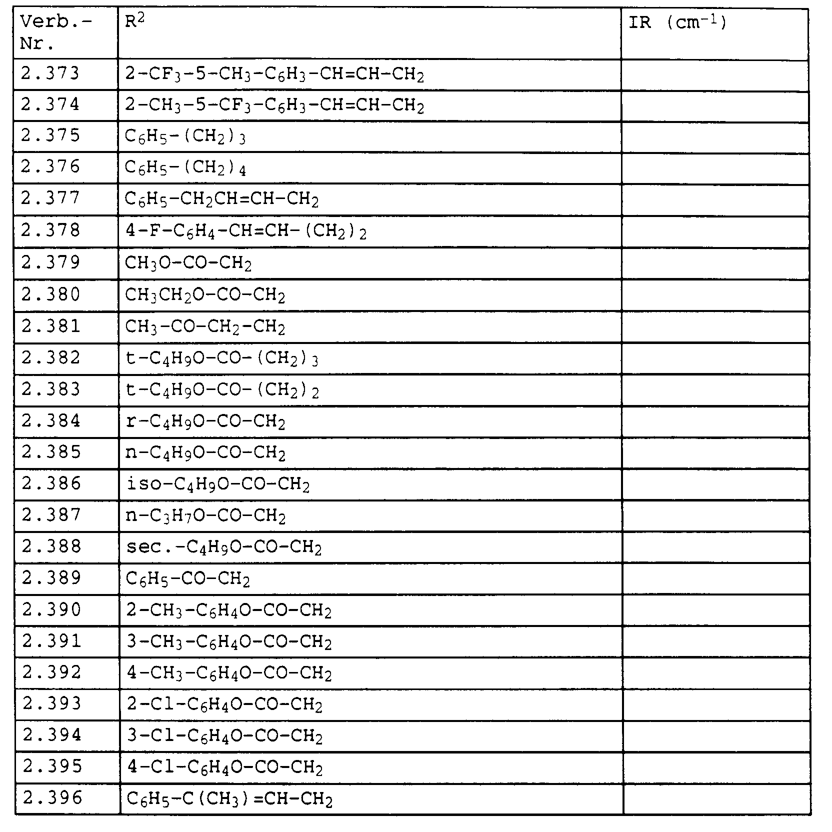

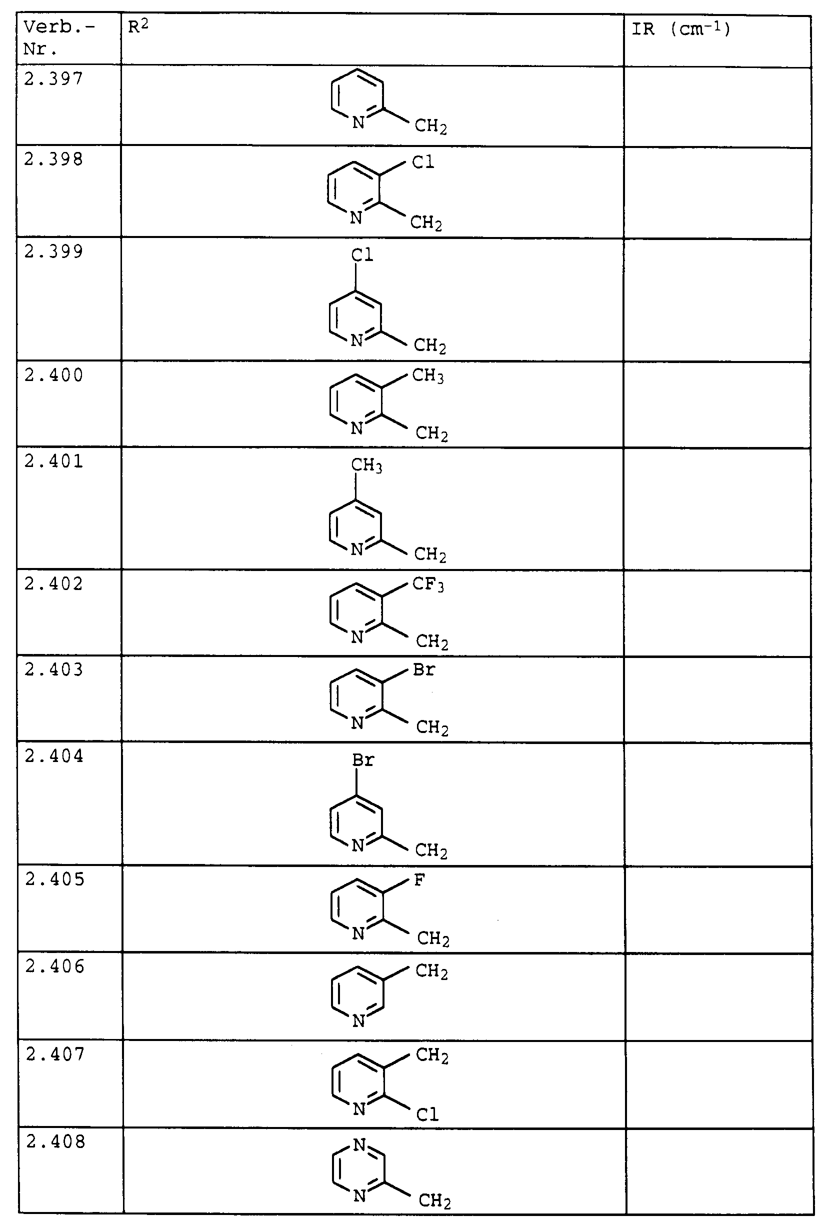

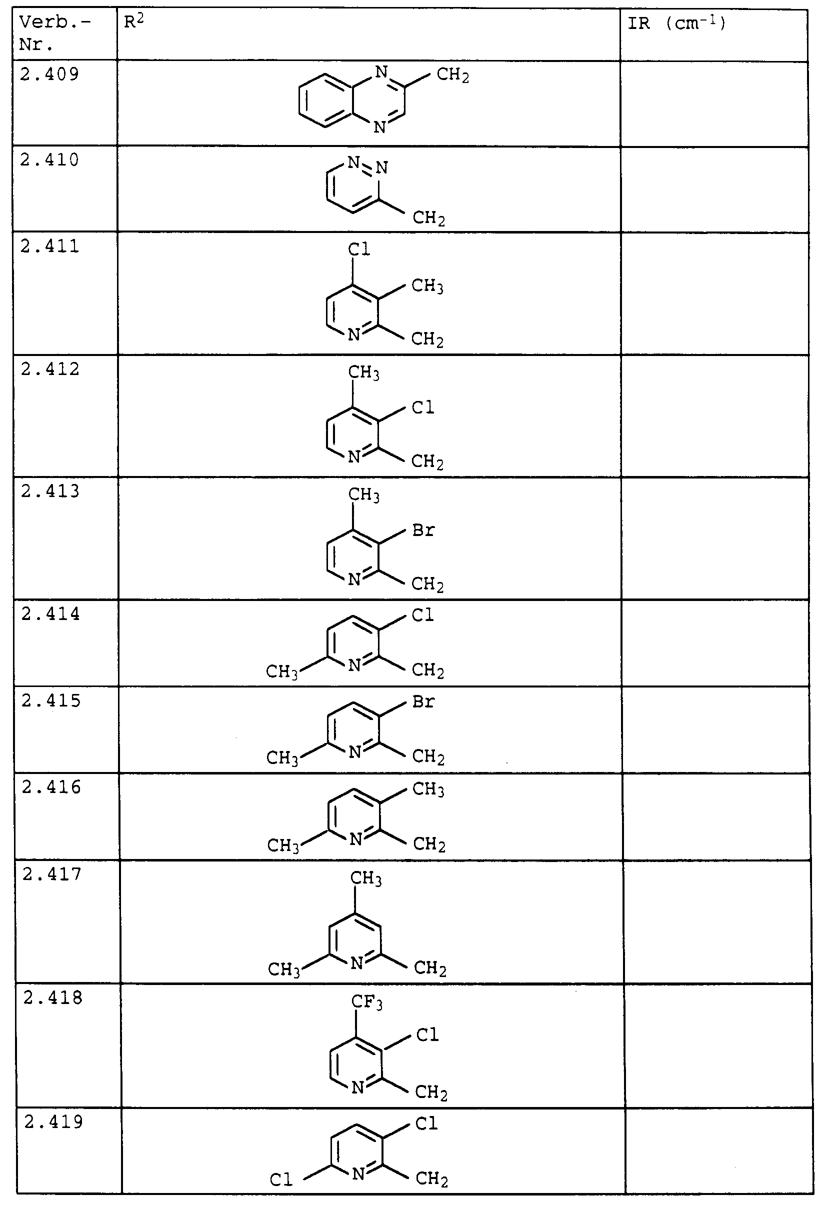

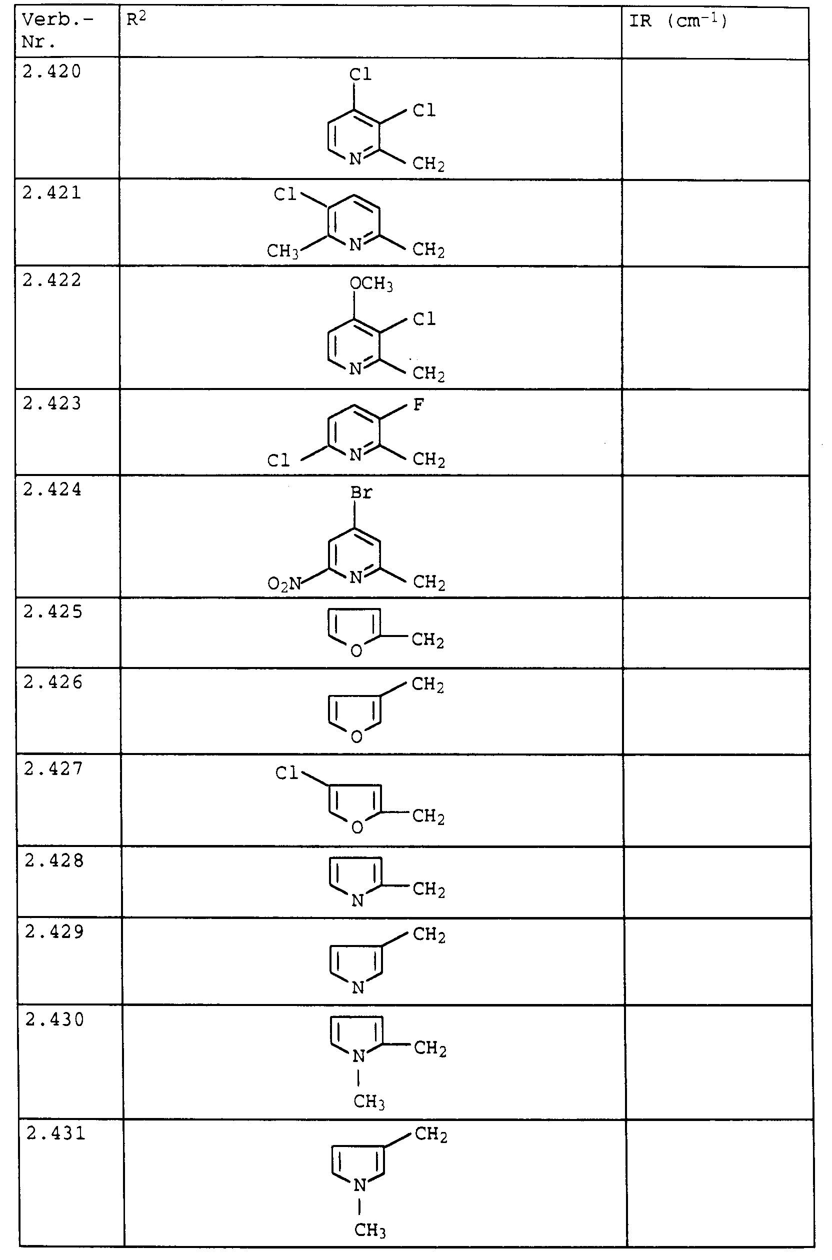

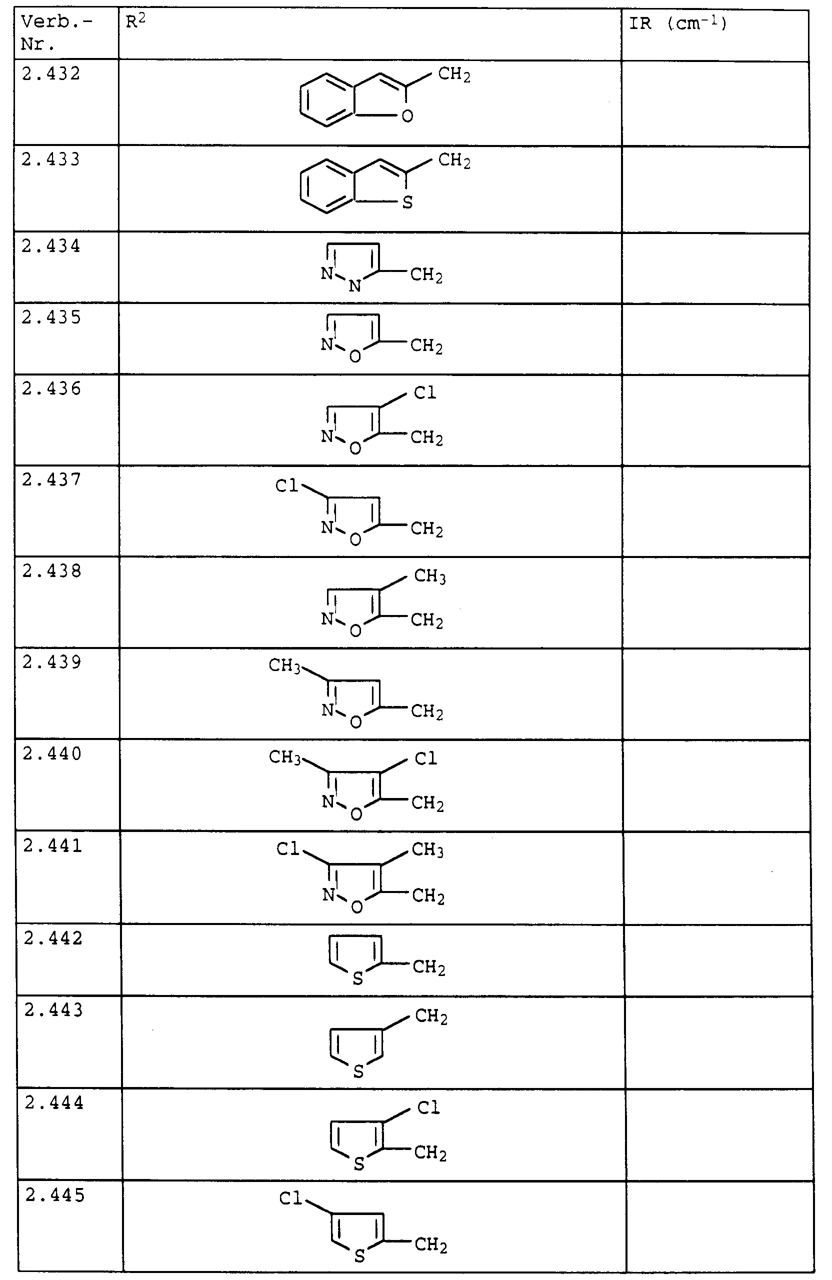

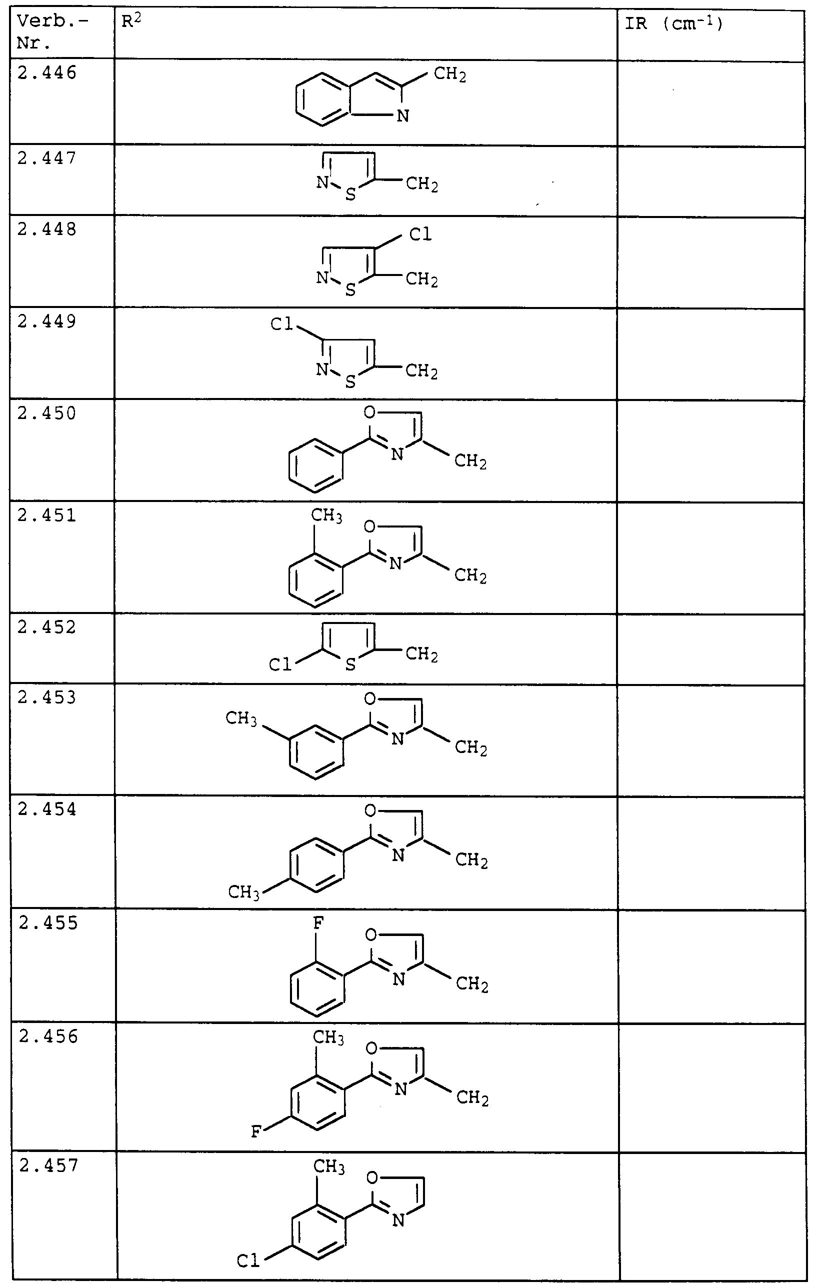

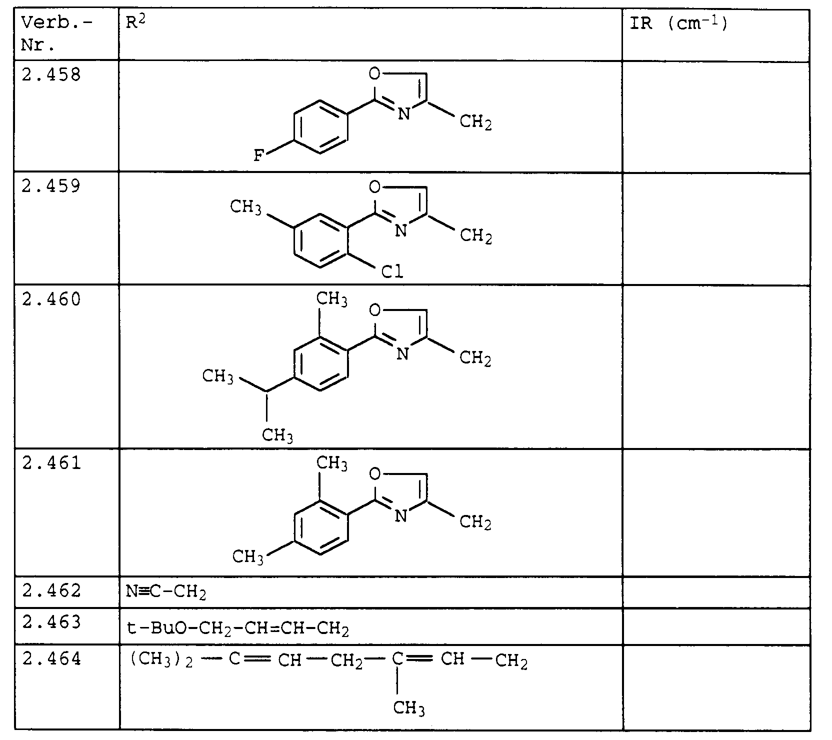

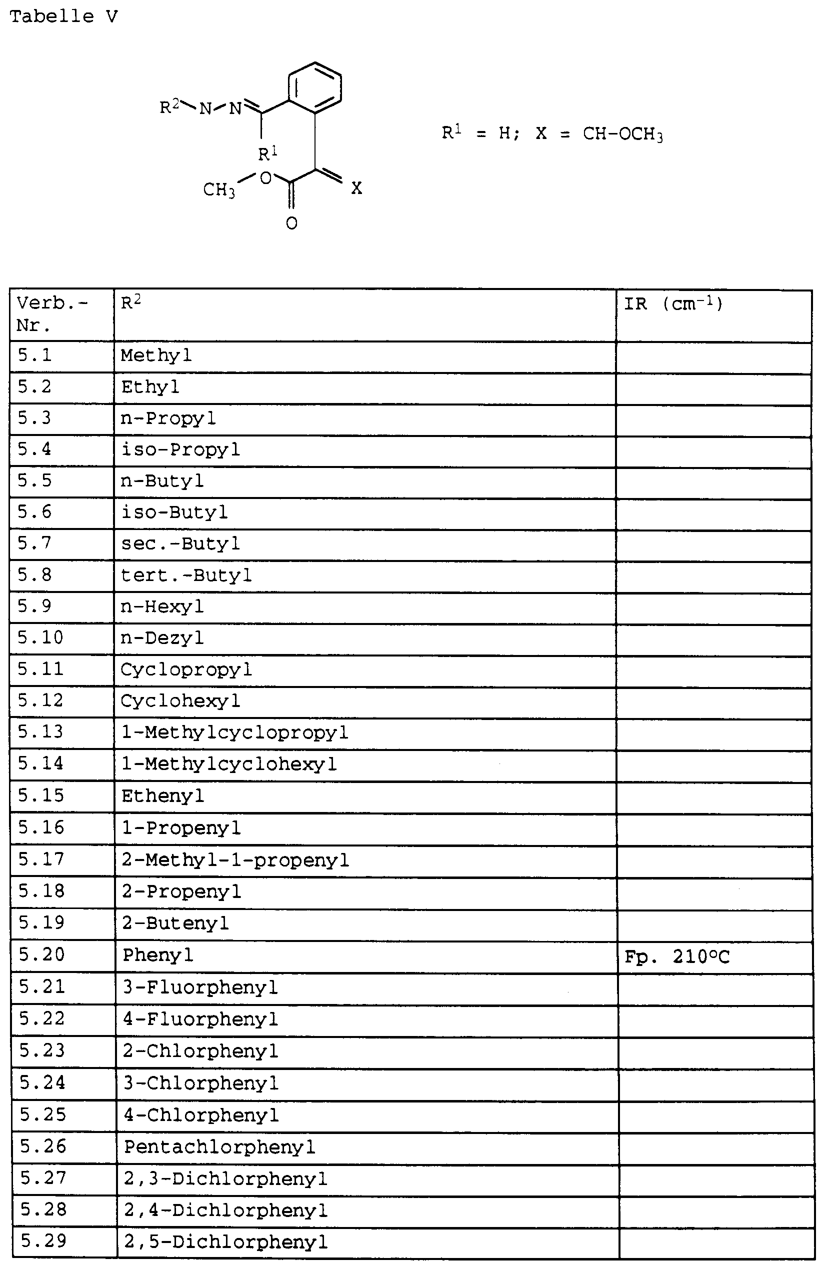

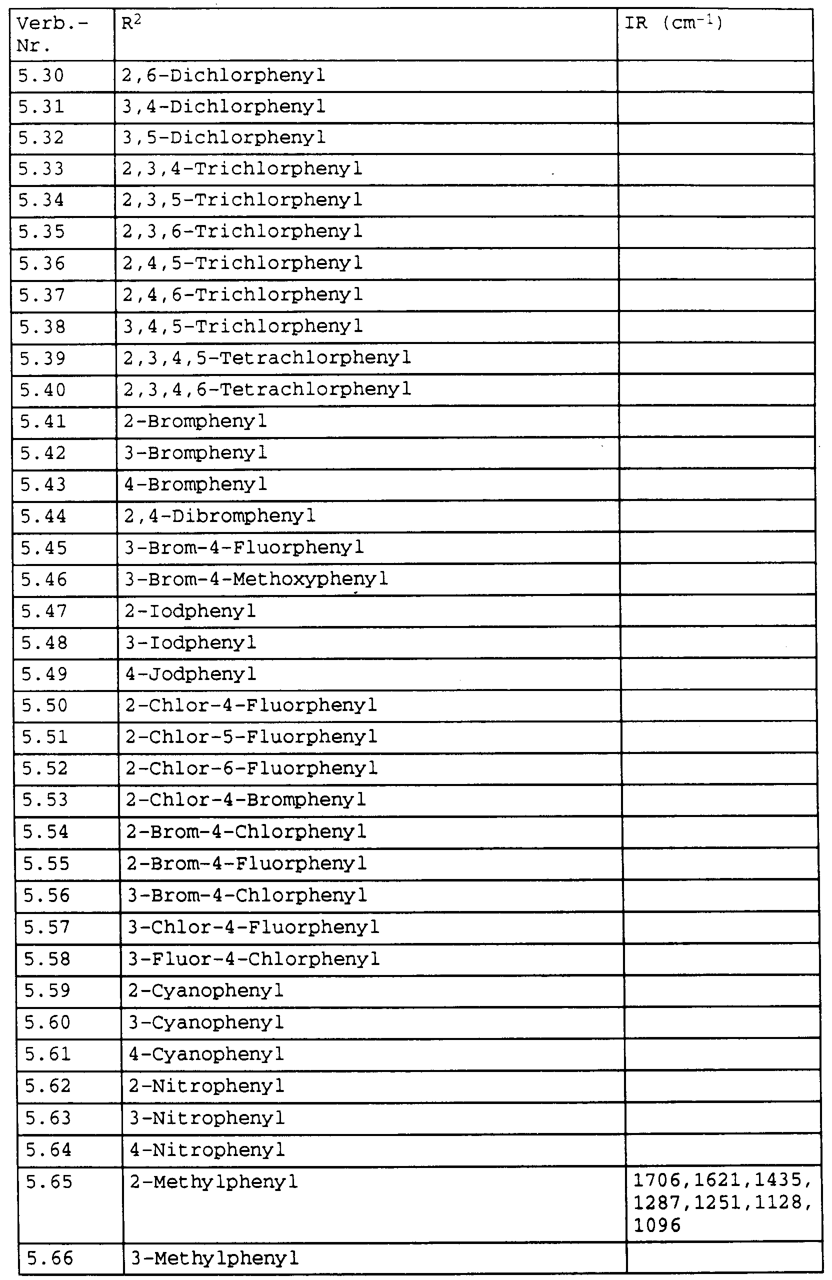

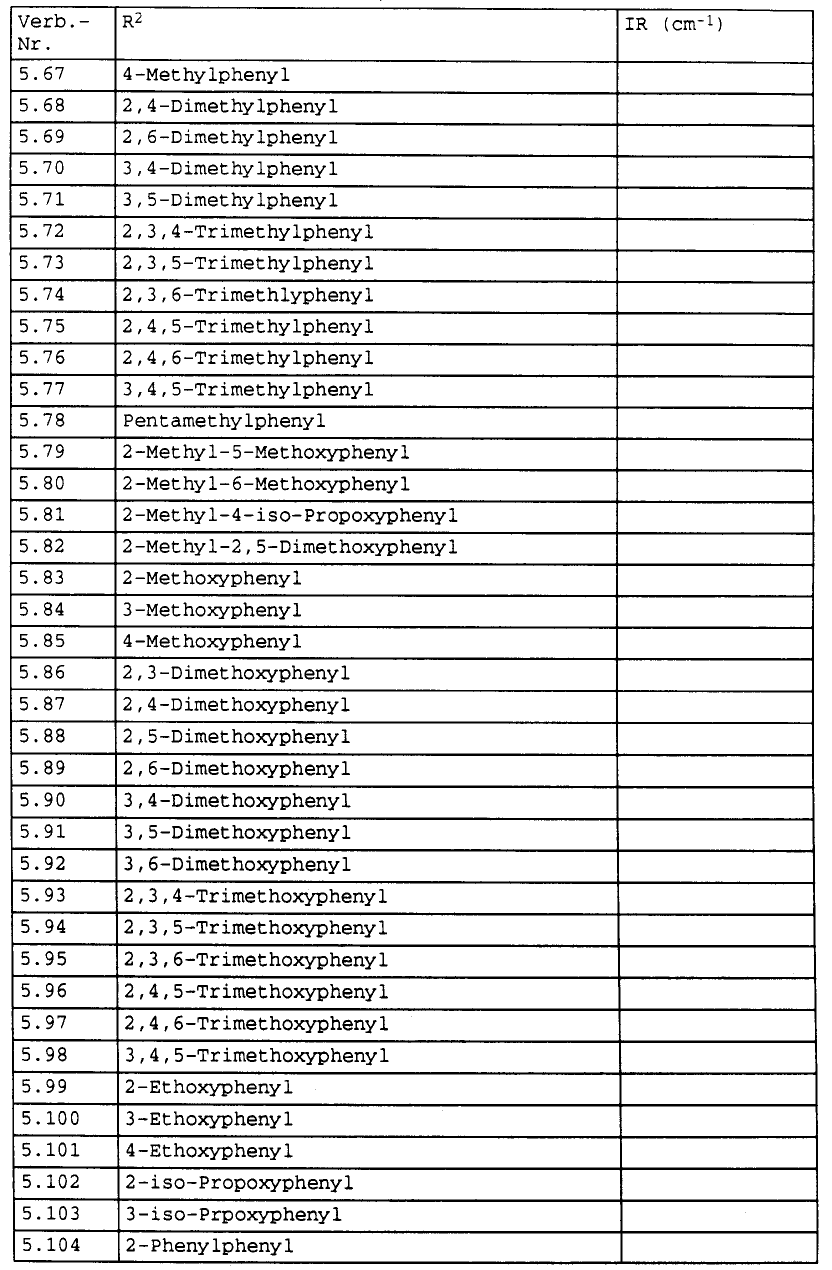

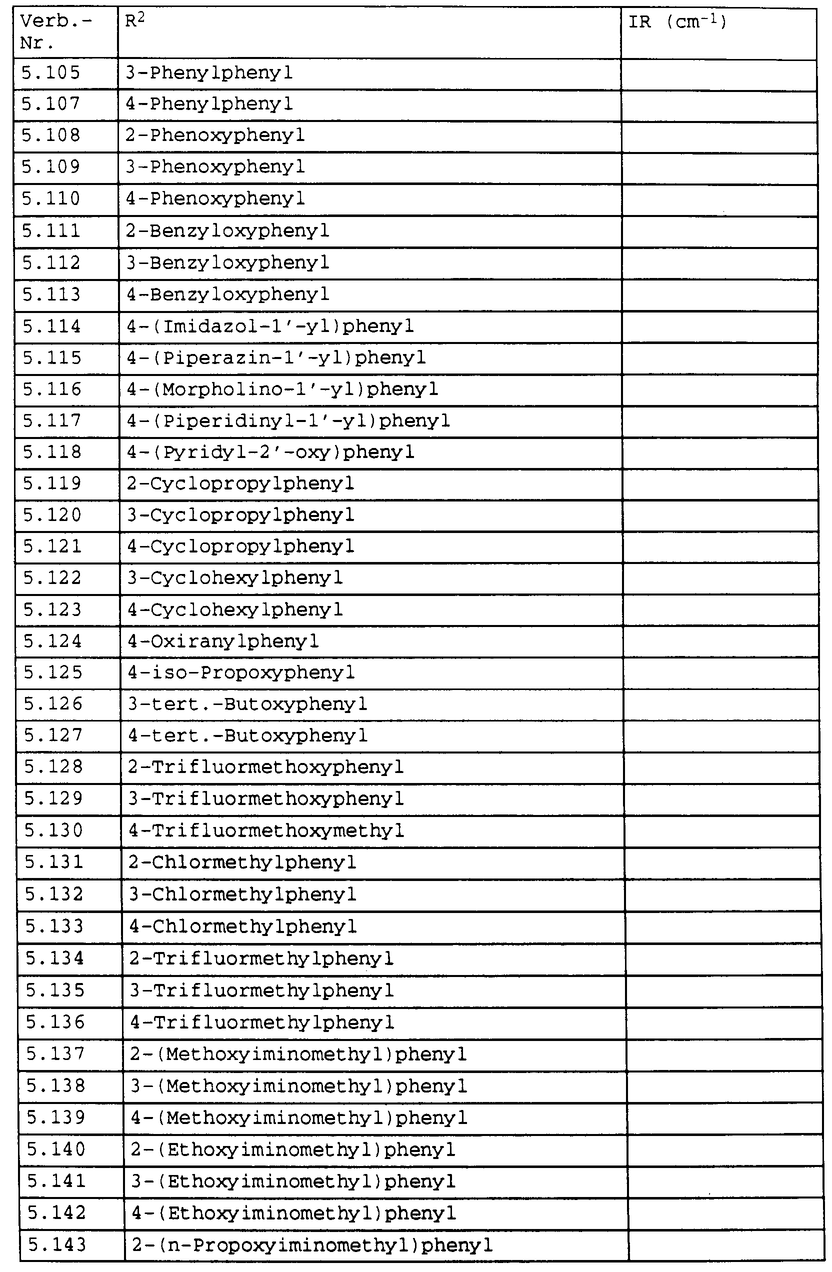

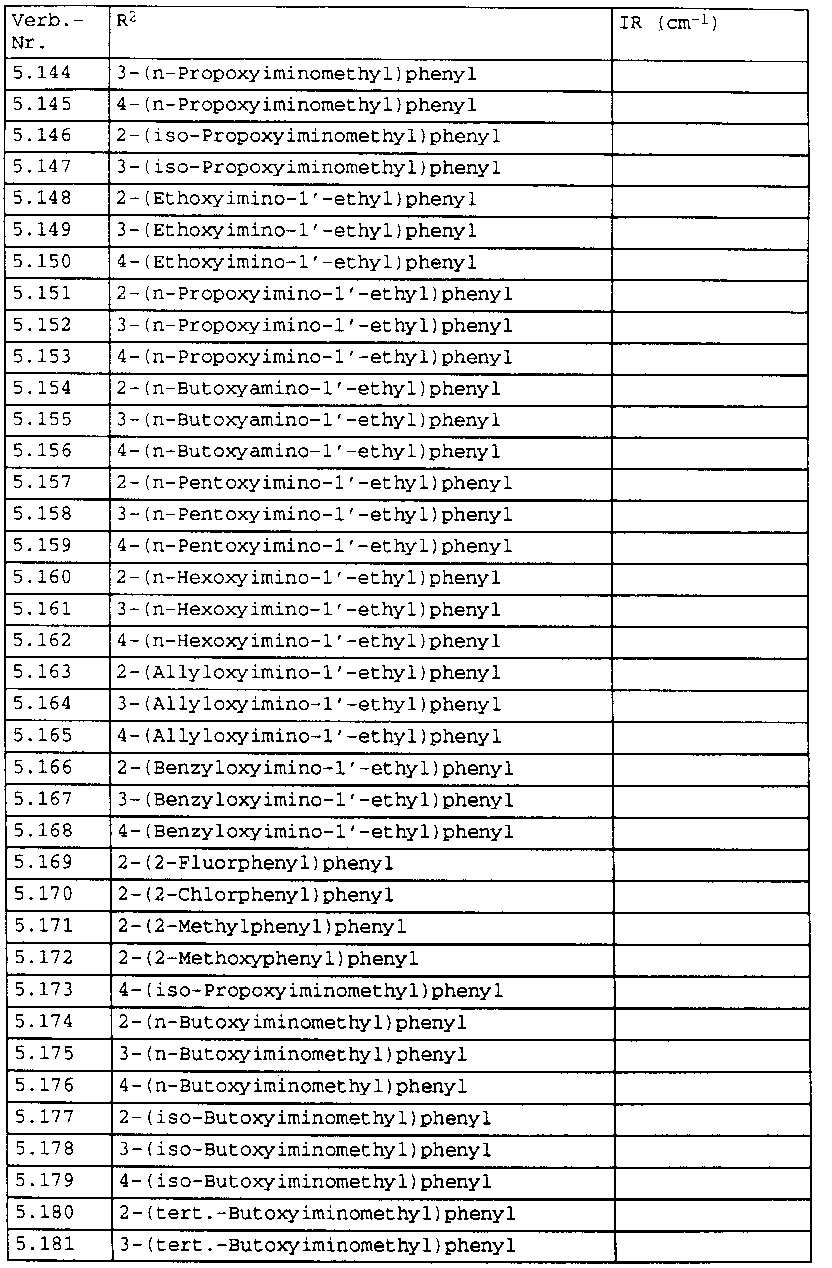

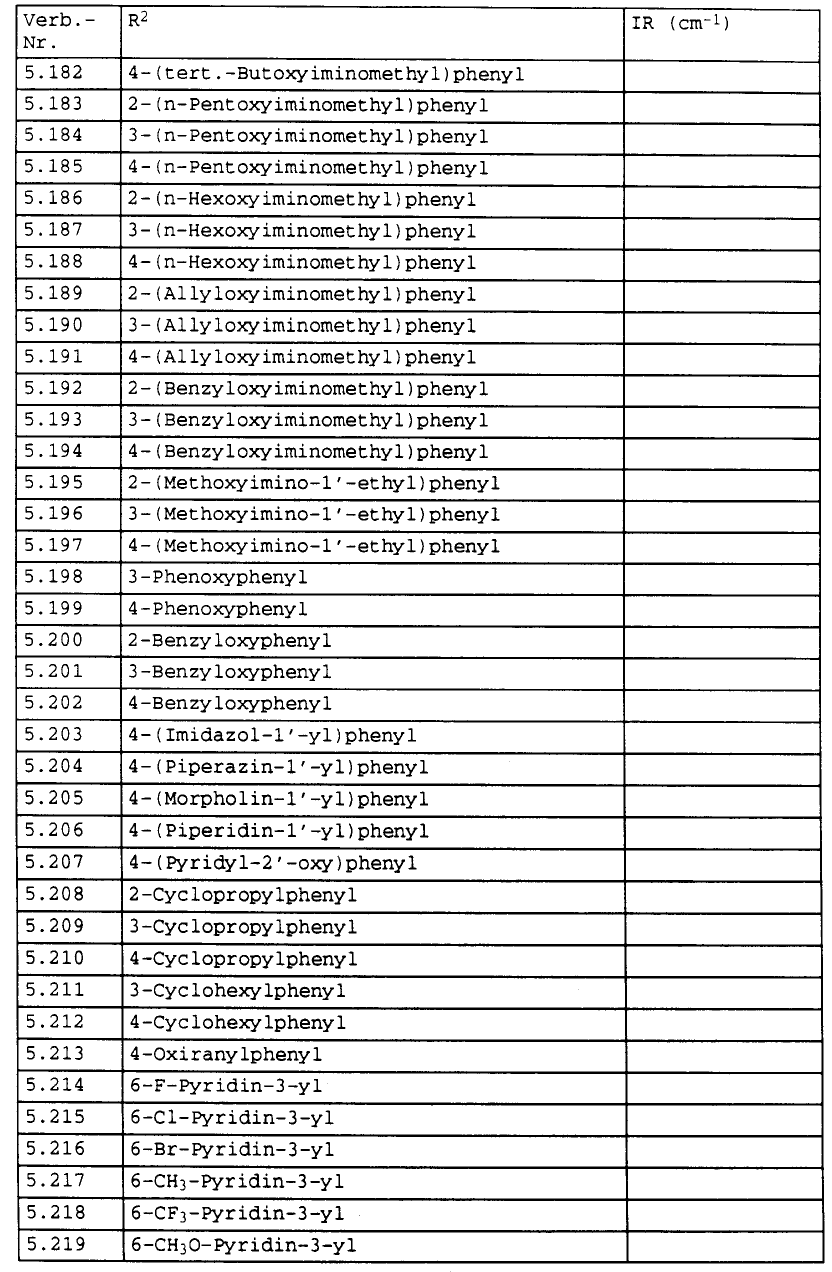

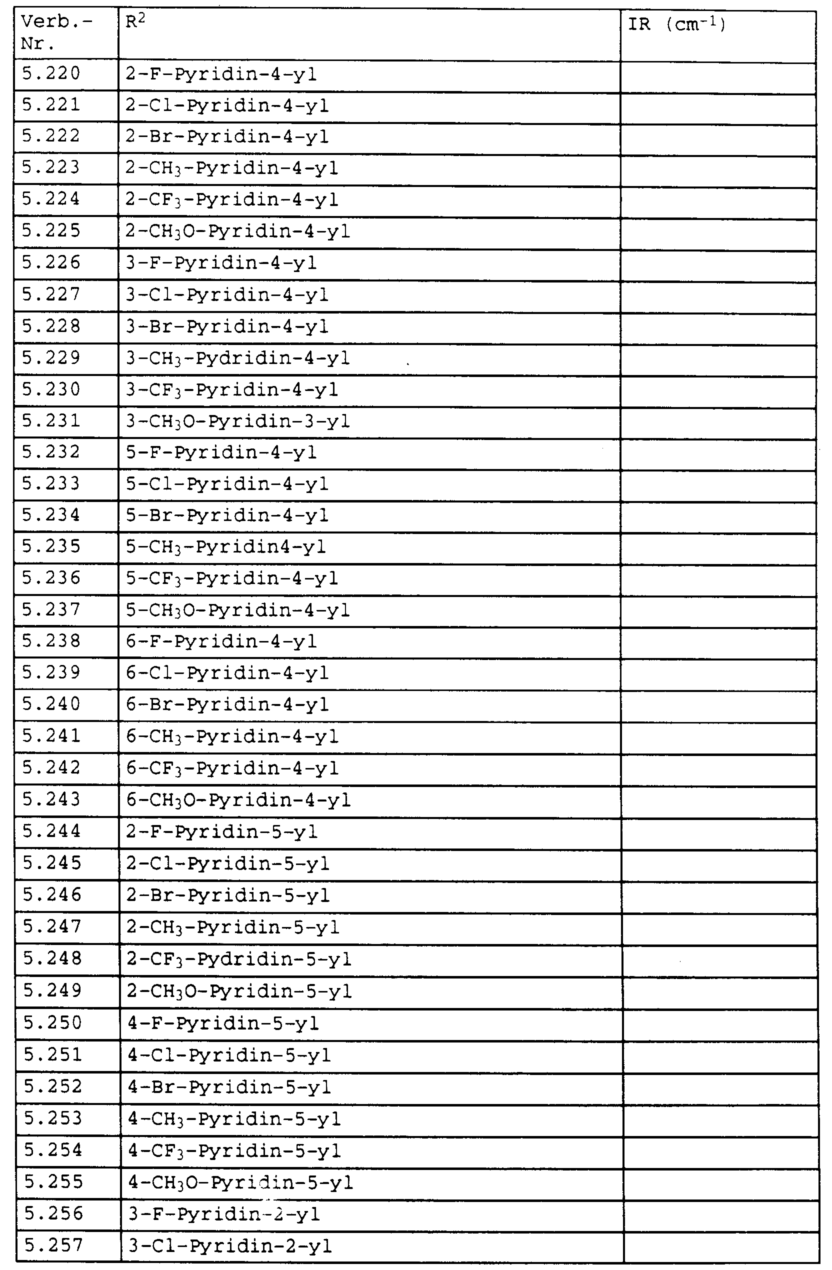

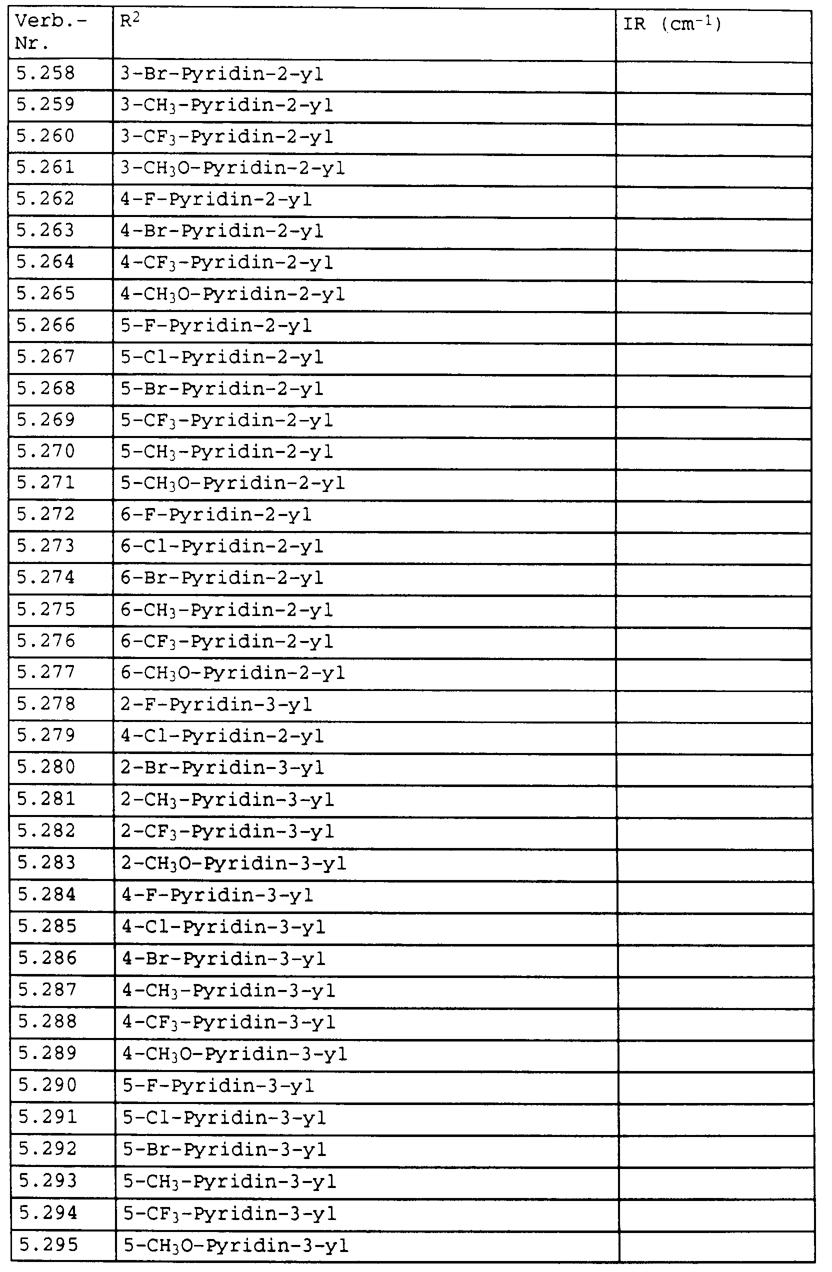

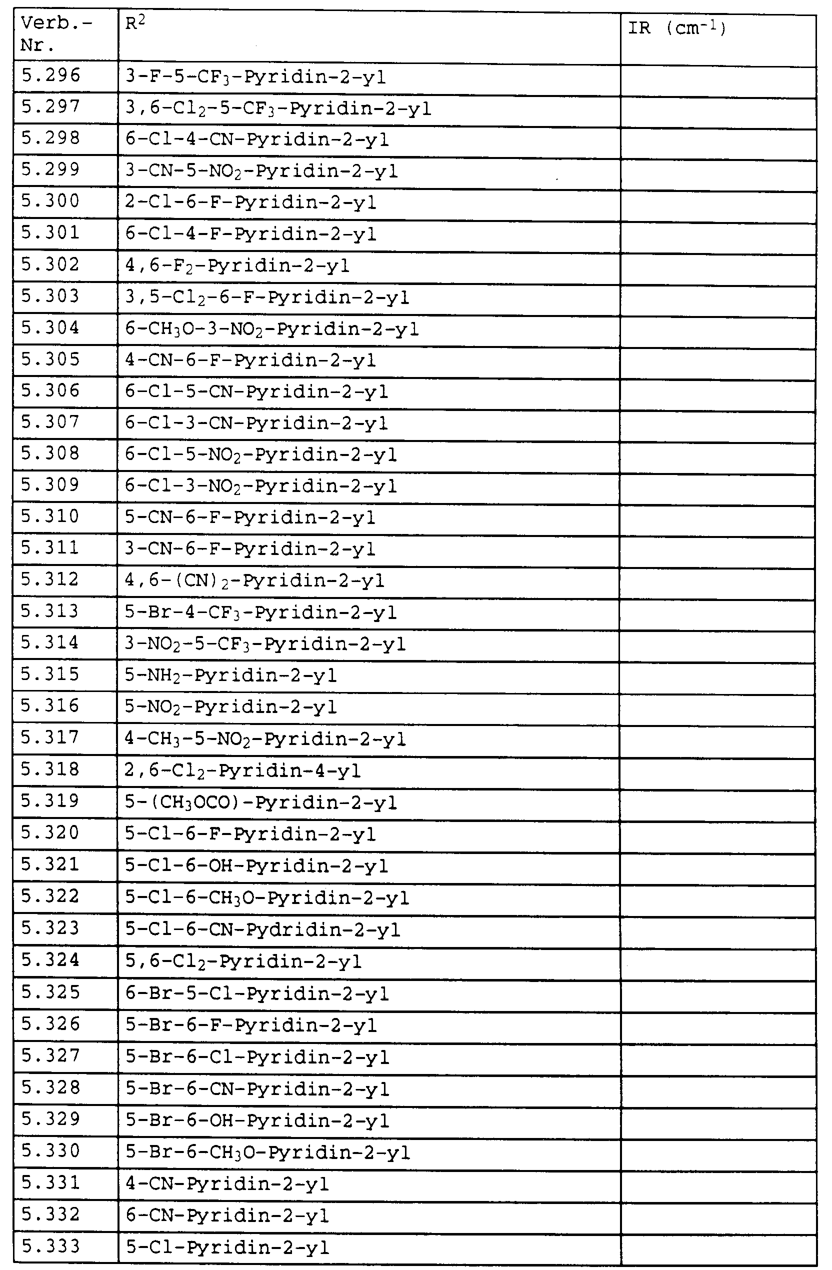

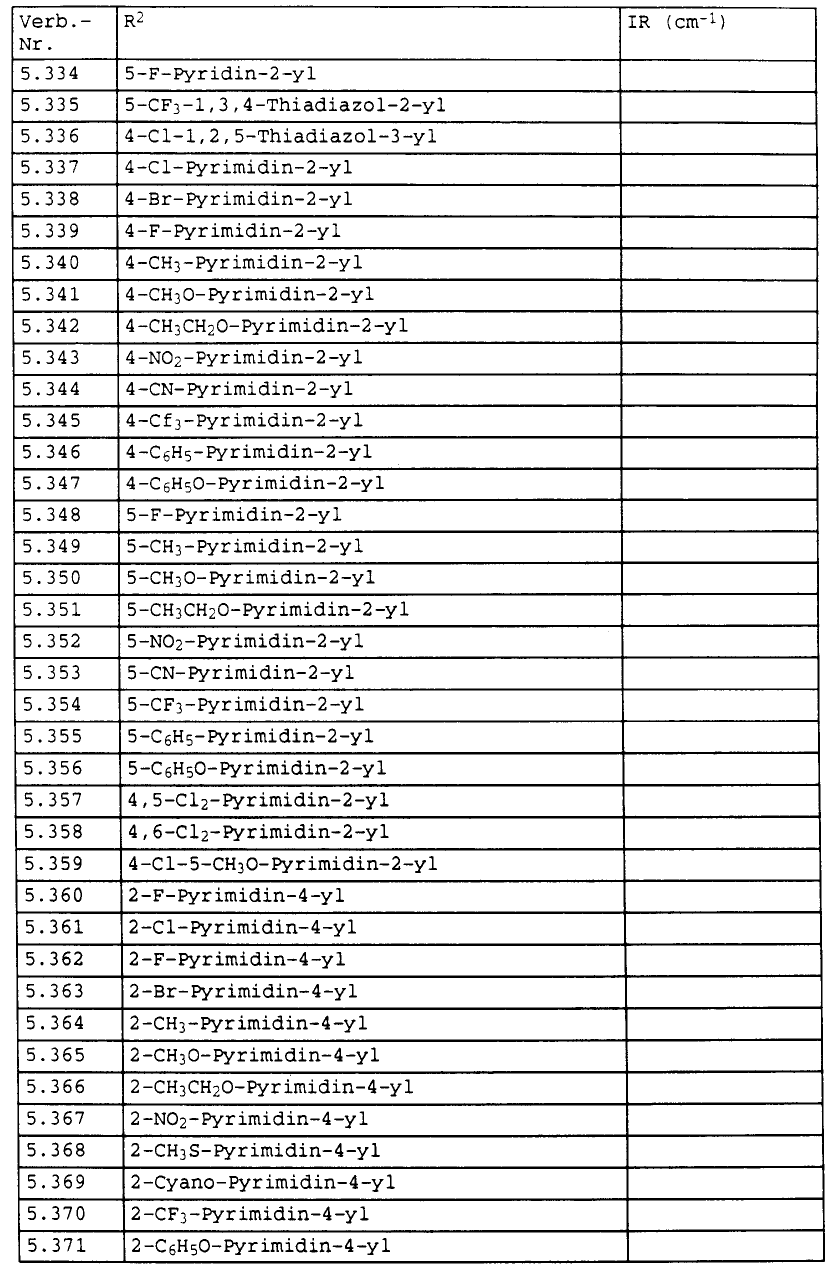

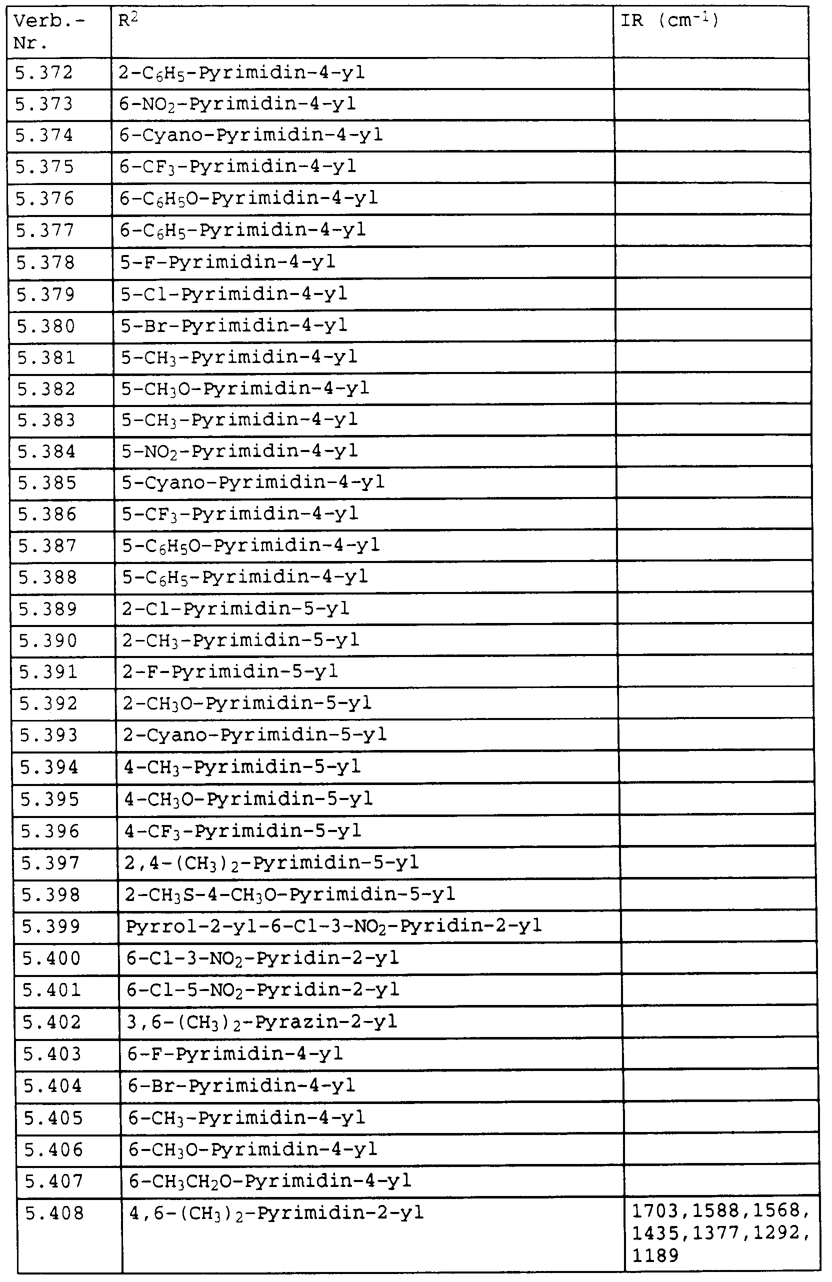

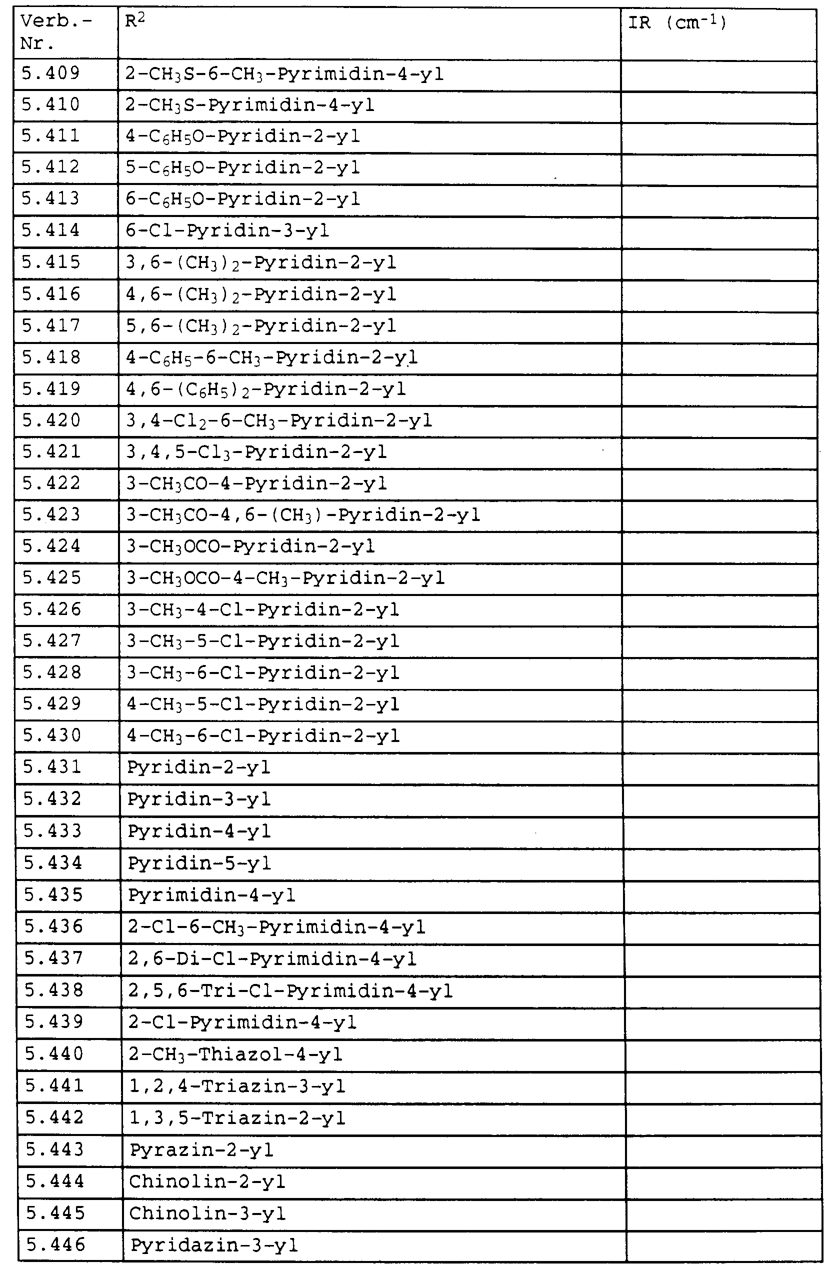

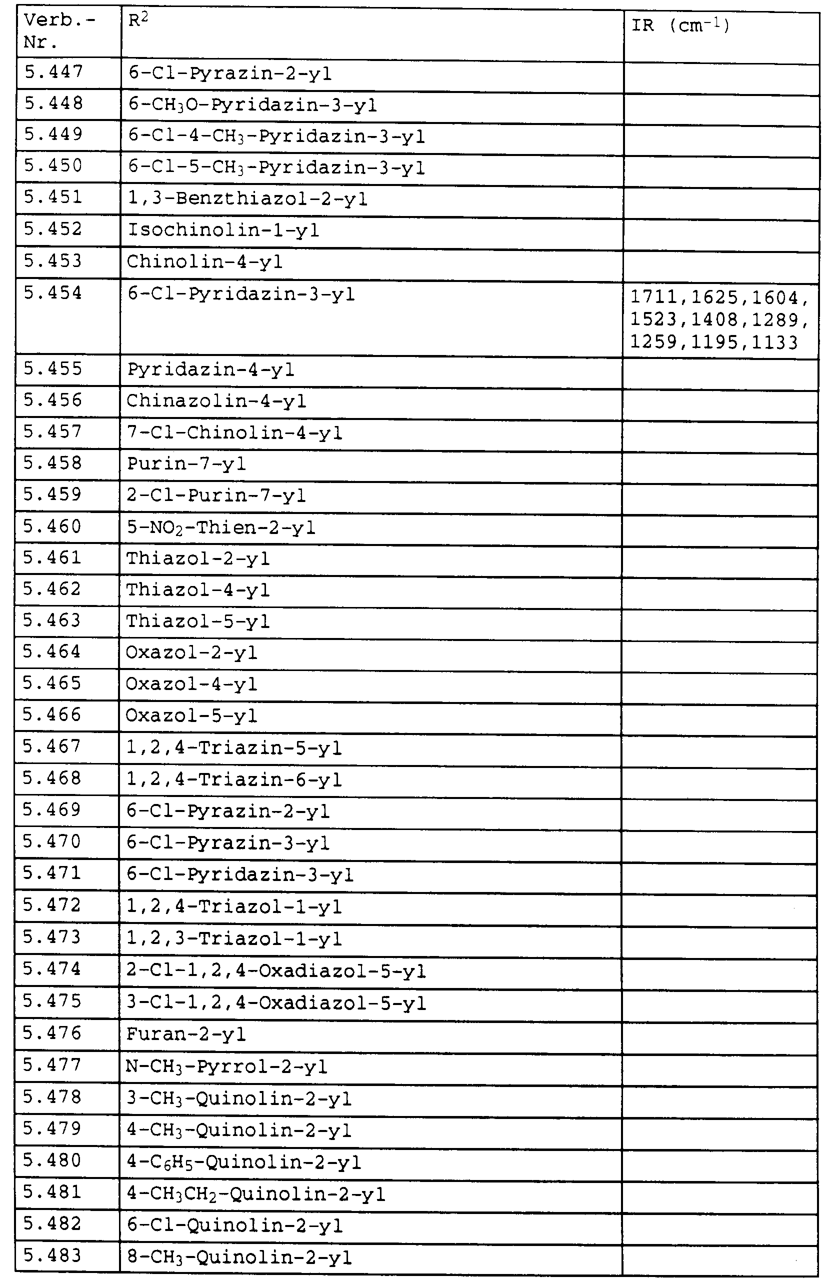

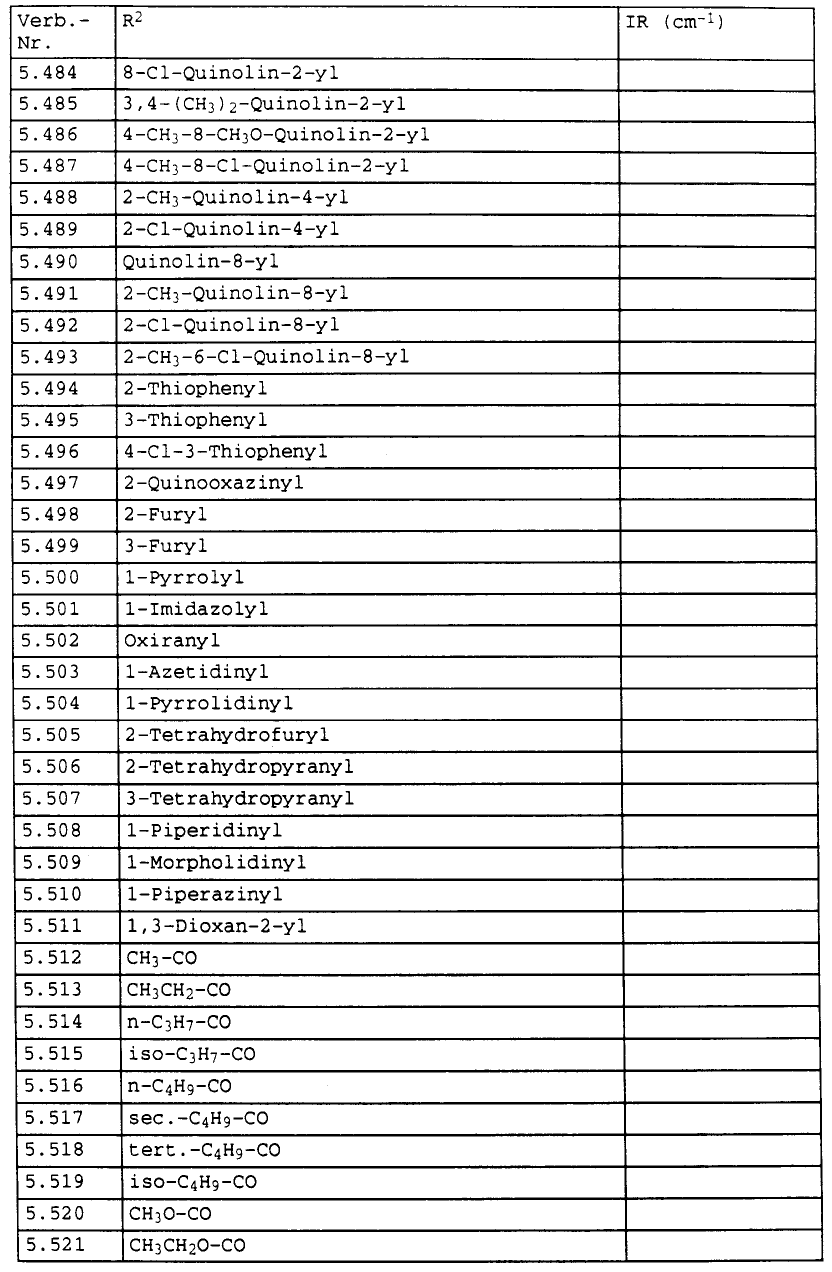

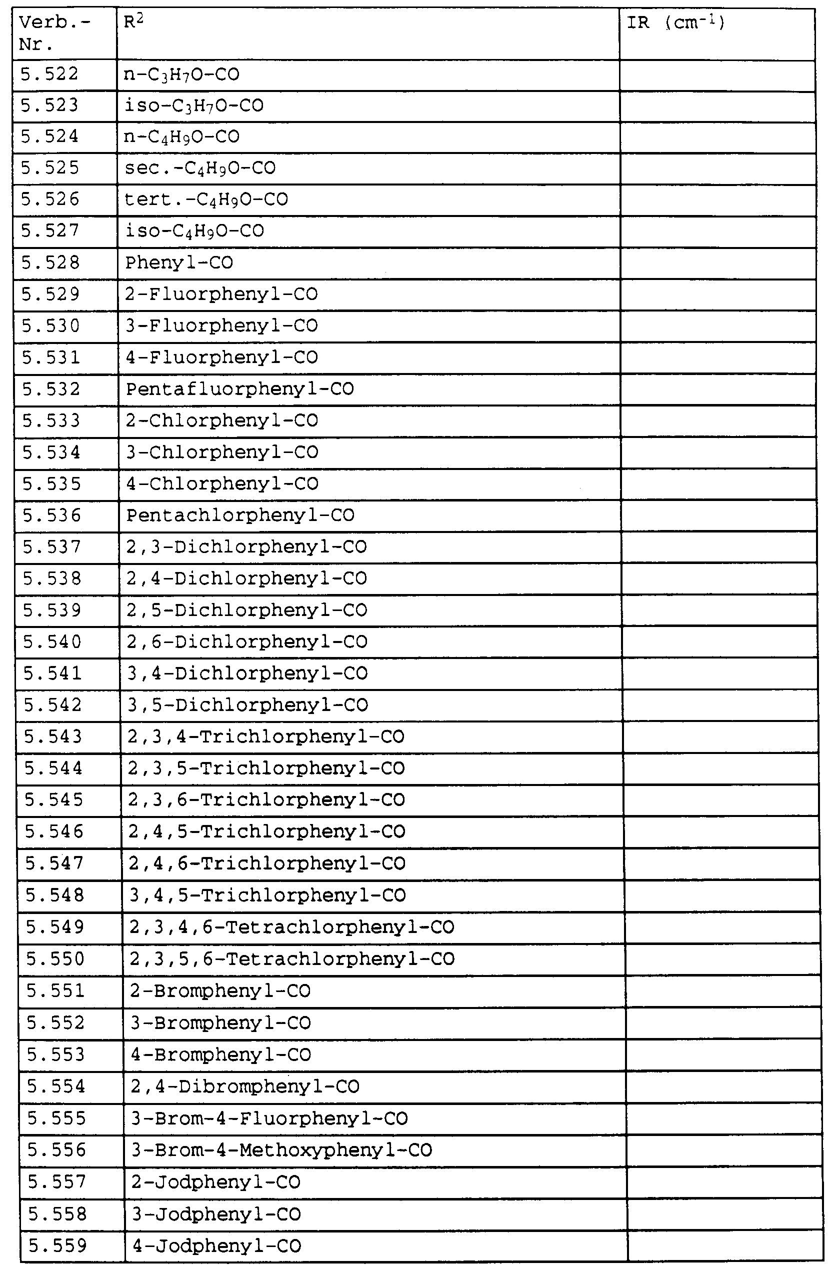

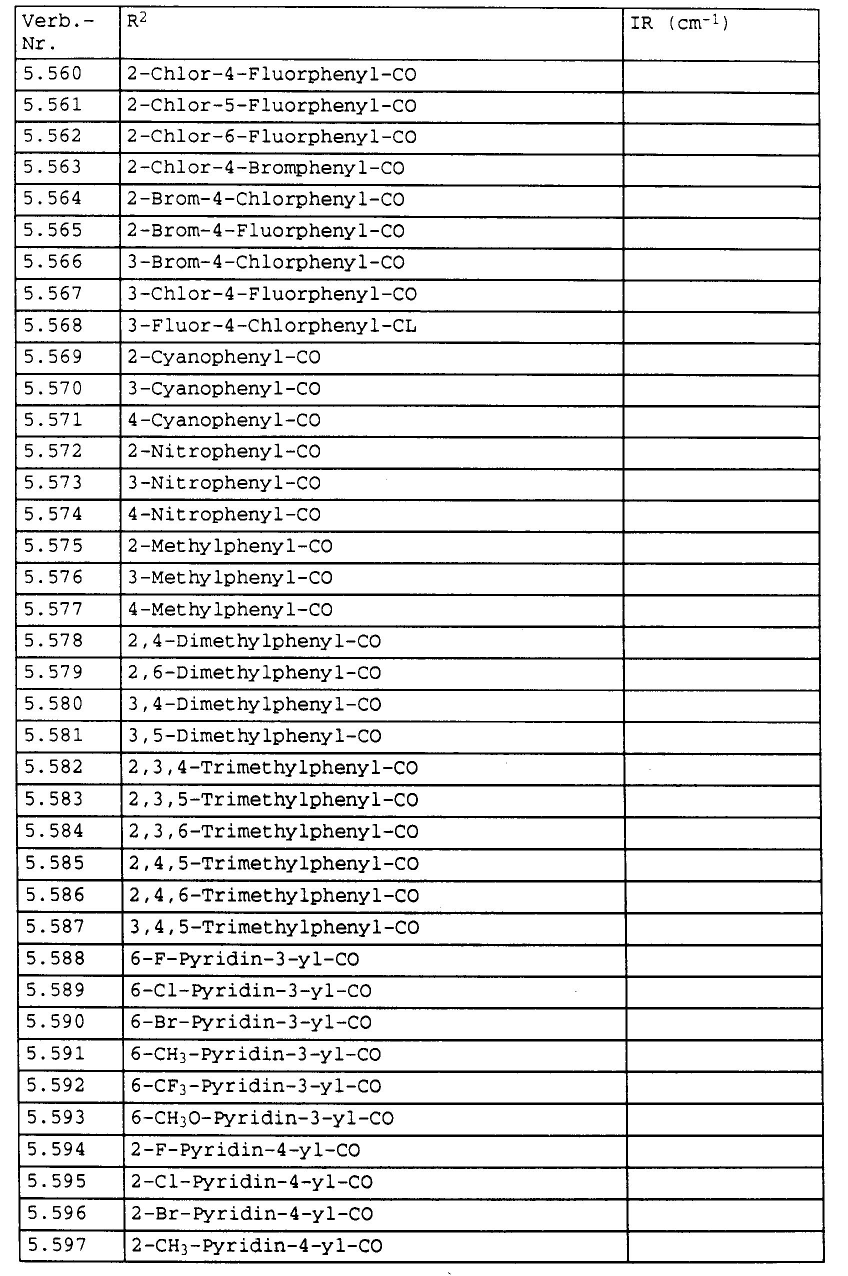

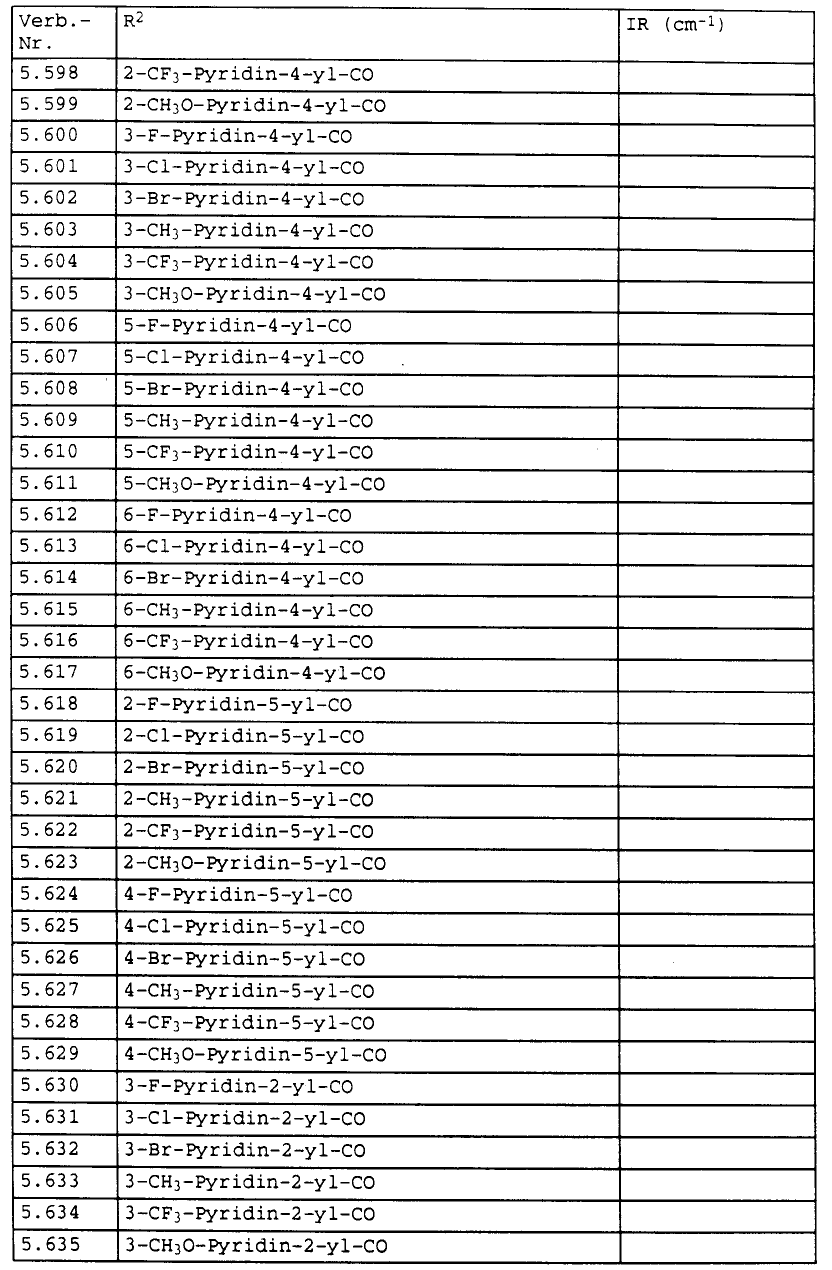

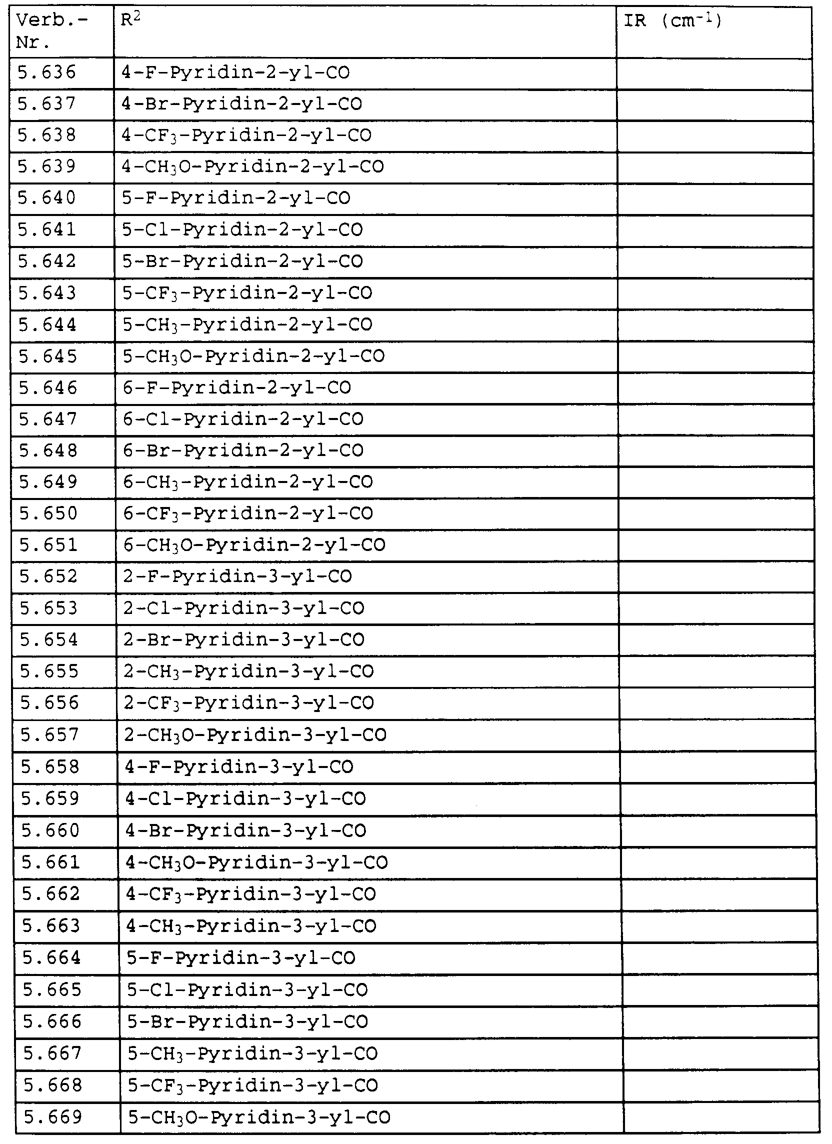

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenyolether, ethoxyliertes Isooctyl-, Octyl- oder Nonylphenol, Alkylphenyol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäure, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen udn Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdgeeren, Reben Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotti.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, wie z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-2,3,4-triazol,
2,3-Dicyano-1,4-dithioanthrachinon,
2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiazidazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis(-1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

## Patentansprüche

1. Verbindungen der Formel I 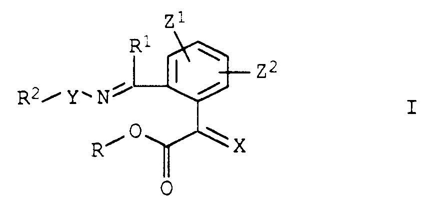 in der die Substituenten folgende Bedeutung haben:
R
Wasserstoff, C₁-C₆-Alkyl;
R¹
Wasserstoff, C₁-C₄-Alkyl, C₁-C₄-Halogenalkyl, ggf. substituiertes Aryl;
Z¹, Z²
gleich oder verschieden sind und Wasserstoff, Halogen, C₁-C₄-Alkoxy, C₂-C₄-Alkenyloxy, Cyano, Nitro, C₁-C₄-Halogenalkyl, C₂-C₄-Halogenalkenyloxy, C₂-C₄-Alkenyl, C₁-C₄-Halogenalkoxy, C₁-C₆-Alkyl oder C₂-C₄-Alkinyl bedeuten;
Y
Sauerstoff (-O-), Stickstoff (-NH-) oder ein durch C₁-C₆-Alkyl (R³) substituierter Stickstoff (-N(R³)-);
R²
Wasserstoff, ggf. substituiertes Alkyl, ggf. substituiertes Cycloalkyl, ggf. substituiertes Alkenyl, ggf. substituiertes Aryl, ggf. substituiertes Alkinyl, ggf. substituiertes Cycloalkenyl, ggf. substituiertes Arylalkyl, ggf. substituiertes Aryloxyalkyl, ggf. substituiertes Heteroaryl, ggf. substituiertes Heteroarylalkyl, ggf. substituiertes Arylalkenyl, ggf. substituiertes Heteroarylalkenyl, ggf. substituiertes Heterocyclyl, ggf. substituiertes Heterocyclyloxy, ggf. substituiertes Arylcarbonyl, ggf. substituiertes Heteroarylcarbonyl oder C₁-C₄-Alkoxycarbonyl-C₁-C₄-alkyl,
wobei Y und R² einen Ring bilden können, der ggf. substituiert sein kann, sofern Y für -NH- oder -NR³- steht;
X
CH-C₁-C₄-Alkoxy oder CH-C₁-C₄-Alkylthio.

2. Verbindungen der Formel I gemäß Anspruch 1, in der X CHOCH₃ oder CHSCH₃ bedeutet.

3. Fungizid, enthaltend einen inerten Trägerstoff und eine fungizid wirksame Menge einer Verbindungen der allgemeinen Formel I gemäß Anspruch 1.

4. Verfahren zur Bekämpfung von Pilzen, dadurch gekennzeichnet, daß man die Pilze oder die von Pilzbefall bedrohten Materialien, Pflanzen, Saatgut oder den Erdboden mit einer fungizid wirksamen Menge einer Verbindung der allgemeinen Formel I gemäß Anspruch 1 behandelt.

5. Verbindung der Formel II

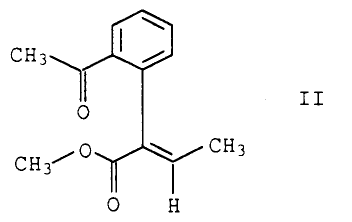

6. Verfahren zur Herstellung von Verbindungen der allgemeinen Formel I gemäß Anspruch 1, dadurch gekennzeichnet, daß man eine Carbonylverbindung der allgemeinen Formel 7 mit einem substituierten Hydroxylamin der Formel 15 oder mit einem substituierten Hydrazin der Formel 16 oder mit einem Säureadditionssalz der Verbindungen 15 oder 16 umsetzt. 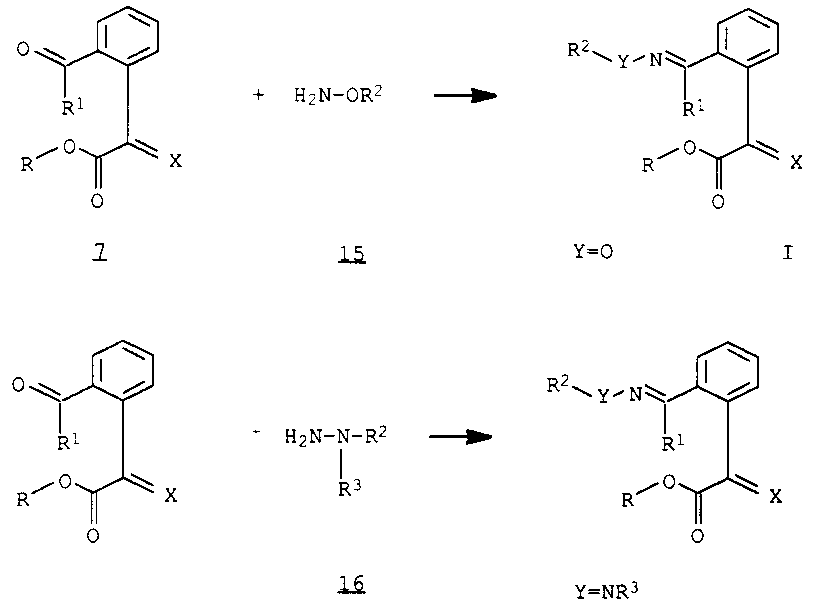 R³ hat die oben angegebene Bedeutung.

## Claims

1. A compound of the formula I 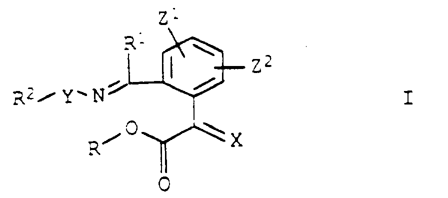 where the substituents have the following meanings:
R is
hydrogen, C₁-C₆-alkyl;
R¹ is
hydrogen, C₁-C₄-alkyl, C₁-C₄-haloalkyl, unsubstituted or substituted aryl;
Z¹, Z² are
identical or different and are hydrogen, halogen, C₁-C₄-alkoxy, C₂-C₄-alkenyloxy, cyano, nitro, C₁-C₄-haloalkyl, C₂-C₄-haloalkenyloxy, C₂-C₄-alkenyl, C₁-C₄-haloalkoxy, C₁-C₆-alkyl or C₂-C₄-alkynyl;
Y is
oxygen (-O-), nitrogen (-NH-) or C₁-C₆-alkyl-substituted ((R³) -substituted) nitrogen (-N(R³)-);
R² is
hydrogen, unsubstituted or substituted alkyl, unsubstituted or substituted cycloalkyl, unsubstituted or substituted alkenyl, unsubstituted or substituted aryl, unsubstituted or substituted alkynyl, unsubstituted or substituted cycloalkenyl, unsubstituted or substituted arylalkyl, unsubstituted or substituted aryloxyalkyl, unsubstituted or substituted heteroaryl, unsubstituted or substituted heteroarylalkyl, unsubstituted or substituted arylalkenyl, unsubstituted or substituted heteroarylalkenyl, unsubstituted or substituted heterocyclyl, unsubstituted or substituted heterocyclyloxy, unsubstituted or substituted arylcarbonyl, unsubstituted or substituted heteroarylcarbonyl or C₁-C₄-alkoxycarbonyl-C₁-C₄-alkyl,
it being possible for Y and R² to form a ring which can be unsubstituted or substituted if Y is -NH- or -NR³-; and
X is
CH-C₁-C₄-alkoxy or CH-C₁-C₄-alkylthio.

2. A compound of the formula I as claimed in claim 1 where X is CHOCH₃ or CHSCH₃.

3. A fungicide comprising an inert carrier and a fungicidally active amount of a compound of the general formula I as claimed in claim 1.

4. A method of controlling fungi, which comprises treating the fungi, or the materials, plants, seed or the soil at risk of fungal infection, with a fungicidally active amount of a compound of the general formula I as claimed in claim 1.

5. The compound of the formula II

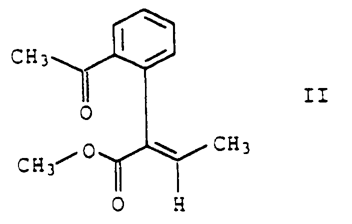

6. A process for the preparation of a compound of the general formula I as claimed in claim 1, which comprises reacting a carbonyl compound of the general formula 7 with a substituted hydroxylamine of the formula 15 or with a substituted hydrazine of the formula 16 or with an acid addition salt of the compound 15 or 16. 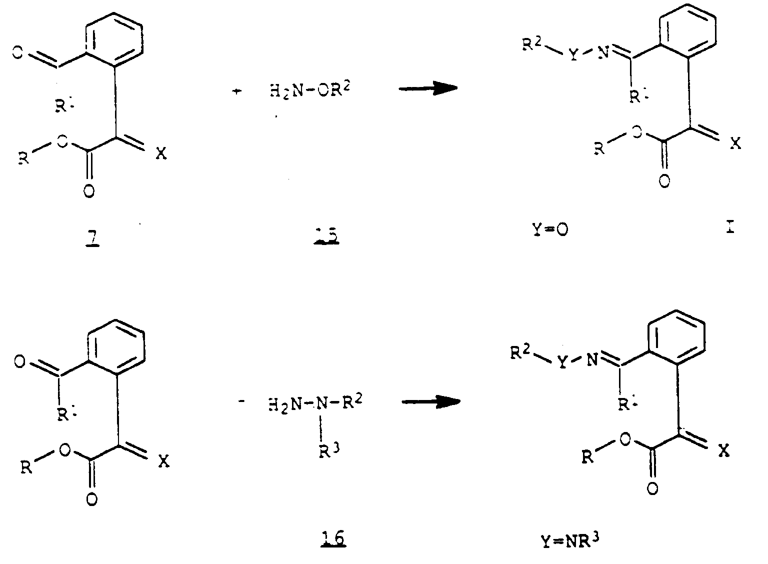 R³ has the meaning indicated above.

## Revendications

1. Composés de formule I 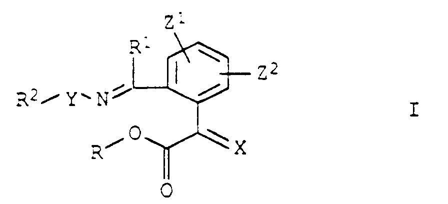 dans laquelle les symboles ont les significations suivantes :
R
l'hydrogène, un groupe alkyle en C1-C6 ;
R¹
l'hydrogène, un groupe alkyle en C1-C4, halogénoalkyle en C1-C4, aryle éventuellement substitué ;
Z¹, Z²
ayant les significations identiques ou différentes, représentent chacun l'hydrogène, un halogène, un groupe alcoxy en C1-C4, alcényloxy en C2-C4, cyano, nitro, halogénoalkyle en C1-C4, halogénoalcényloxy en C2-C4, alcényle en C2-C4, halogénoalcoxy en C1-C4, alkyle en C1-C6 ou alcynyle en C2-C4 ;
Y
l'oxygène (-O-), l'azote (-NH-) ou un atome d'azote substitué par un groupe alkyle en C1-C6 (R³) (-N(R³)-) ;
R²
l'hydrogène, un groupe alkyle éventuellement substitué, cycloalkyle éventuellement substitué, alcényle éventuellement substitué, aryle éventuellement substitué, alcynyle éventuellement substitué, cycloalcényle éventuellement substitué, arylalkyle éventuellement substitué, aryloxyalkyle éventuellement substitué, hétéroaryle éventuellement substitué, hétéroarylalkyle éventuellement substitué, arylalcényle éventuellement substitué, hétéroarylalcényle éventuellement substitué, hétérocyclyle éventuellement substitué, hétérocyclyloxy éventuellement substitué, arylcarbonyle éventuellement substitué, hétéroarylcarbonyle éventuellement substitué ou (alcoxy en C1-C4)carbonylalkyle en C1-C4,
Y et R² pouvant également former un cycle lui-même éventuellement substitué lorsque Y représente -NH- ou -NR³- ;
X
un groupe -CH-alcoxy en C1-C4 ou CH-alkylthio en C1-C4.

2. Composés de formule I selon la revendication 1, dans laquelle X représente CHOCH₃ ou CHSCH₃.

3. Produit fongicide contenant un véhicule inerte et une quantité fongicide efficace d'un composé de formule générale I selon la revendication 1.

4. Procédé pour combattre les mycètes, caractérisé par le fait que l'on traite les mycètes ou les matières, végétaux, semences ou sols menacés d'une attaque par les mycètes par une quantité fongicide efficace d'un composé de formule générale I selon la revendication 1.

5. Composé de formule II

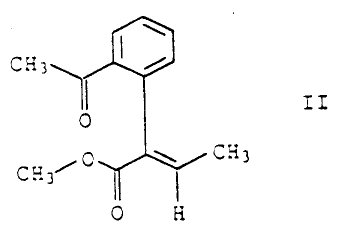

6. Procédé de préparation des composés de formule générale I selon la revendication 1, caractérisé par le fait que l'on fait réagir un dérivé carbonylé de formule générale 7 avec une hydroxylamine substituée de formule 15 ou avec une hydrazine substituée de formule 16 ou avec un sel d'un composé 15 ou 16 par addition avec un acide : 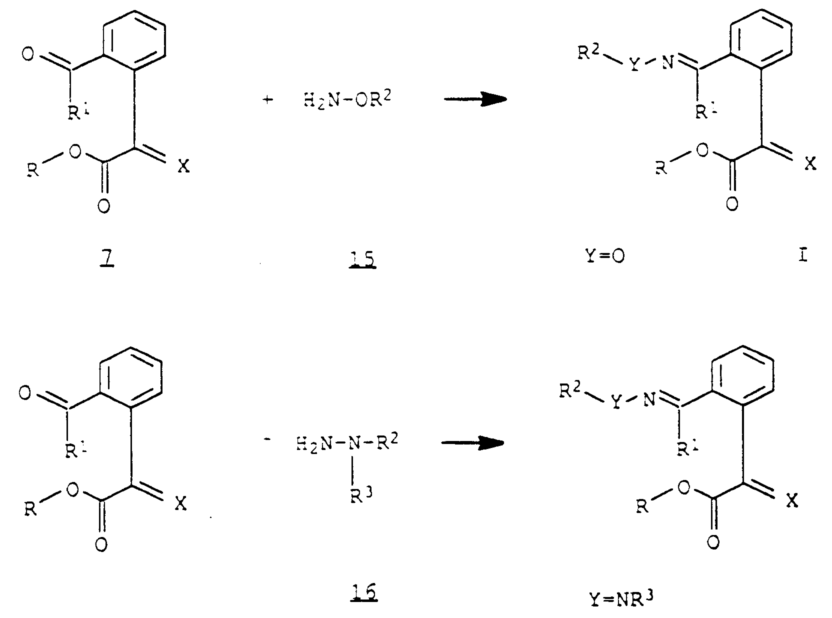 R³ ayant les significations indiquées ci-dessus.
